(19) Europäisches Patentamt
European Patent Office
Office européen des brevets

(11) Veröffentlichungsnummer: **0 334 813 B1**

# (12) EUROPÄISCHE PATENTSCHRIFT

(45) Veröffentlichungstag der Patentschrift: **02.02.94**

(21) Anmeldenummer: **89810209.0**

(22) Anmeldetag: **16.03.89**

(51) Int. Cl.⁵: **C07D 213/79**, C07D 213/83, C07D 401/12, C07D 405/12, C07D 409/12, A01N 43/40, A01N 43/50, A01N 43/647

## (54) Mittel zum Schutz von Pflanzen gegen Krankheiten.

(30) Priorität: **25.03.88 CH 1140/88**

(43) Veröffentlichungstag der Anmeldung:
**27.09.89 Patentblatt 89/39**

(45) Bekanntmachung des Hinweises auf die Patenterteilung:
**02.02.94 Patentblatt 94/05**

(84) Benannte Vertragsstaaten:
**AT BE CH DE ES FR GB GR IT LI LU NL SE**

(56) Entgegenhaltungen:
**EP-A- 0 053 307**
**EP-A- 0 268 775**
**DE-A- 2 459 051**
**US-A- 4 137 067**

(73) Patentinhaber: **CIBA-GEIGY AG**
**Klybeckstrasse 141**
**CH-4002 Basel(CH)**

(72) Erfinder: **Zondler, Helmut, Dr.**
**Oberwilerstrasse 49**
**CH-4103 Bottmingen(CH)**
Erfinder: **Kunz, Walter, Dr.**
**Buchenstrasse 9**
**CH-4104 Oberwil(CH)**

**EP 0 334 813 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte Isonicotinsäureester der nachstehenden Formel I. Die Erfindung betrifft ferner die Herstellung dieser Substanzen sowie die als Wirkstoff mindestens eine dieser Verbindungen enthaltenden Mittel. Die Erfindung betrifft darüber hinaus die Herstellung der genannten Mittel sowie die Verwendung der Wirkstoffe oder der Mittel zum Schutz von Pflanzen gegen den Befall durch schädliche Mikroorganismen, beispielsweise pflanzenschädigende Pilze, Bakterien und Viren.

Die erfindungsgemässen Verbindungen entsprechen der allgemeinen Formel I

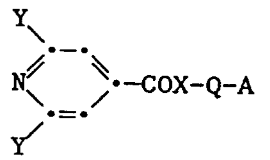

(I)

in welcher bedeuten:

Y Halogen;

X Sauerstoff oder Schwefel;

Q $C_1$-$C_3$-Alkylen, Propenylen, mit R ein- oder zweifach substituiertes $C_1$-$C_3$-Alkylen oder mit R ein- oder zweifach substituiertes Propenylen.

R $C_1$-$C_4$-Alkyl, $C_1$-$C_4$-Haloalkyl mit 1 bis 3 Halogenatomen, Cyano, $C_2$-$C_5$-Alkoxycarbonyl, $C_3$-$C_6$-Cycloalkyl, Phenyl oder mit $C_1$-$C_4$-Alkyl, $C_1$-$C_4$-Alkoxy, Trifluormethyl, Trichlormethyl, Nitro oder Cyano substituiertes Phenyl, oder Benzoyl oder mit $C_1$-$C_4$-Alkyl, $C_1$-$C_4$-Alkoxy, Trifluormethyl, Trichlormethyl, Nitro oder Cyano substituiertes Benzoyl;

A Phenyl, Biphenyl, Phenoxyphenyl, Naphthyl, Pyridyl, Furyl, Thienyl, Imidazolyl oder Triazolyl, wobei diese Reste unsubstituiert oder mit Halogen, $C_1$-$C_4$-Alkyl, $C_1$-$C_3$-Alkoxy, Trifluormethyl, Nitro oder Cyano substituiert sein können; oder R ist p-Chlorphenyl, wenn Y = Chlor, X = Sauerstoff, Q = Methylen und A = 3,4-Dimethoxyphenyl sind; mit der Massgabe, 1.) dass falls A Imidazolyl oder Triazolyl darstellt, R nicht Phenyl oder Benzoyl sein darf, und 2.) dass für A und für R in Q zusammen maximal 3 Ringe stehen dürfen.

Halogen selbst oder als Bestandteil eines anderen Substituenten bedeutet Fluor, Chlor, Brom oder Jod, vorzugsweise Chlor oder Brom, und insbesondere bevorzugt Chlor.

Unter Alkyl selbst oder als Bestandteil eines anderen Substituenten sind gerad- und verzweigtkettige Alkyle zu verstehen. Sie stellen je nach Zahl der angegebenen Kohlenstoffatome beispielsweise folgende Gruppen dar: Methyl, Ethyl sowie die Isomeren von Propyl oder Butyl, wie z.B. Isopropyl, Isobutyl, tert.-Butyl oder sek.-Butyl.

Die Erfindung bezieht sich insbesondere auf Verbindungen der Formel I, worin bedeuten:

Y Halogen;

X Sauerstoff oder Schwefel;

Q $C_1$-$C_3$-Alkylen, Propenylen, mit R ein- oder zweifach substituiertes $C_1$-$C_3$-Alkylen oder mit R ein- oder zweifach substituiertes Propenylen.

R $C_1$-$C_4$-Alkyl, $C_1$-$C_4$-Haloalkyl mit 1 bis 3 Halogenatomen, Cyano, $C_2$-$C_5$-Alkoxycarbonyl, $C_3$-$C_6$-Cycloalkyl, Phenyl oder mit $C_1$-$C_4$-Alkyl, $C_1$-$C_4$-Alkoxy, Trifluormethyl, Trichlormethyl, Nitro oder Cyano substituiertes Phenyl, oder Benzoyl oder mit $C_1$-$C_4$-Alkyl, $C_1$-$C_4$-Alkoxy, Trifluormethyl, Trichlormethyl, Nitro oder Cyano substituiertes Benzoyl;

A Phenyl, Biphenyl, Phenoxyphenyl, Naphthyl, Pyridyl, Furyl, Thienyl, Imidazolyl oder Triazolyl, wobei diese Reste unsubstituiert oder mit Halogen, $C_1$-$C_4$-Alkyl, $C_1$-$C_3$-Alkoxy, Trifluormethyl, Nitro oder Cyano substituiert sein können; mit der Massgabe, 1.) dass falls A Imidazolyl oder Triazolyl darstellt, R nicht Phenyl oder Benzoyl sein darf, und 2.) dass für A und für R in Q zusammen maximal 3 Ringe stehen dürfen.

Die unter Formel I als substituiert definierten cyclischen Reste sind vorzugsweise ein- bis dreifach substituiert.

Aufgrund ihrer besonderen pflanzenschützenden Eigenschaften lassen sich die Verbindungen der Formel I in folgende Gruppen einteilen.

1. Verbindungen der Formel I, worin bedeuten:

Y gleichzeitig Chlor oder Brom;

X Sauerstoff;

Q Methylen oder mit R substituiertes Methylen;

R $C_1$-$C_3$-Alkyl, Phenyl oder mit Halogen oder Methoxy substituiertes Phenyl;
A Phenyl, mit Halogen substituiertes Phenyl, oder Pyridyl, Furyl, Thienyl, Imidazolyl oder Triazolyl.
2. Verbindungen der Formel I, worin bedeuten:
Y Chlor;
X Sauerstoff;
Q mit R substituiertes Methylen;
R Methyl, Ethyl, Phenyl oder 2,4-Dichlorphenyl;
A mit Chlor und/oder Fluor substituiertes Phenyl, insbesondere 2,4-Dichlorphenyl.

Die folgenden Verbindungen zeichnen sich durch besonders vorteilhafte pflanzenschützende Eigenschaften aus:
2,6-Dichlor-isonicotinsäurebenzylester;
2,6-Dichlor-isonicotinsäure-α-methylbenzylester;
2,6-Dichlor-isonicotinsäure-α-ethylbenzylester;
2,6-Dichlor-isonicotinsäure-α-phenylbenzylester;
2,6-Dichlor-isonicotinsäure-α-(4-chlorphenyl)-benzylester.

2,6-Dihaloisonicotinsäure-Derivate sind bereits teilweise bekannt. So sind in der Schweizer Patentschrift Nr. 384 929 und der britischen Patentschrift Nr. 923,387 2,6-Dihaloisonicotinsäurederivate, z.B. die freie Säure und einige ihrer Ester und Salze als Herbizide beschrieben. Ferner sind in der USA-Patentschrift No. 4,137,067 und in der kanadischen Patentschrift No. 1,072,443 2,6-Dichlorisonicotinsäurealkylester als Zwischenprodukte zur Herstellung von als fungizid wirksam beschriebenen Hydrazidderivaten der vorgenannten Isonicotinsäureverbindungen angegeben.

Darüber hinaus sind 2,6-Dihalo-isonicotinsäure-Derivate als Tuberkulostatika bekannt geworden (vgl. Acta. Fac. Pharm. Brun. Bratislav. 4, 65-66 [1962]; Chem. Abst. Vol. 57, 1962, 4769b).

Es wurde nun überraschenderweise gefunden, dass die erfindungsgemässen Verbindungen der Formel I durch ihre Verwendung den Befall von Pflanzen durch schädliche Mikroorganismen verhindern und damit den befallsbedingten Schädigungen der Pflanzen vorbeugen. Für die erfindungsgemässen Wirkstoffe ist charakteristisch, dass der Schutz der Pflanzen sowohl durch direkte Einwirkung auf die pflanzenschädigenden Mirkoorganismen mittels Blattapplikation (direkte Wirkung) oder Bodenapplikation (systemische Wirkung) als auch durch Aktivierung und Stimulierung des pflanzeneigenen Abwehrsystems (Immunisierung) eintreten kann. Der grosse Vorteil der Verbindungen der Formel I besteht darin, dass die Gesunderhaltung der mit diesen Stoffen behandelten Pflanzen auch aus eigener Kraft ohne Einsatz weiterer mikrobizider Substanzen während der Vegetationsperiode gewährleistet werden kann. Demzufolge ist es möglich, durch die Anwendung der erfindungsgemässen Wirkstoffe nachteilige Nebeneffekte, wie sie bei der direkten Parasitenbekämpfung mit chemischen Substanzen z.B. einerseits durch Schädigung der Nutzpflanzen (Phytotoxizität) und andererseits durch Hervorrufung von Resistenzerscheinungen bei den schädlichen Mikroorganismen in Erscheinung treten können, zu vermeiden, was vorteilhafterweise ein völlig ungestörtes Wachstum der Nutzpflanzen zur Folge hat.

Aufgrund der doppelten Wirkungsweise der erfindungsgemässen Verbindungen der Formel I, nämlich einerseits der direkten Bekämpfung der Pflanzenpathogene und andererseits der Erhöhung der allgemeinen Abwehrbereitschaft der mit diesen Wirkstoffen behandelten Pflanzen durch Immunisierung kann ein breit gefächerter Schutz der Pflanzen gegen Krankheiten erzielt werden. Die Anwendung der erfindungsgemässen Wirkstoffe ist deshalb besonders für praktische Bedingungen geeignet. Darüber hinaus bewirkt die den Verbindungen der Formel I zueigene systemische Aktivität, dass sich der Schutzeffekt auch auf zuwachsende Pflanzenteile der behandelten Pflanzen erstreckt.

Die allgemein pflanzenschützende Aktivität der erfindungsgemässen Wirkstoffe ist z.B. gegen die den folgenden Klassen angehörenden phytopathogenen Fungi wirksam: Fungi imperfecti (z.B. Botrytis, Helminthosporium, Fusarium, Septoria, Cercospora und Alternaria); Basidiomyceten (z.B. die Gattungen Hemileia, Rhizoctonia, Puccinia); Ascomyceten (z.B. Venturia, Podosphaera, Erysiphe, Monilinia, Uncinula).

Darüber hinaus können die Wirkstoffe besonders vorteilhaft gegen folgende Schadorganismen eingesetzt werden:
Pilze, wie z.B. Oomyceten (z.B. Plasmopara viticola, Phytophthora infestans, Peronospora tabacina, Pseudoperonospora), Fungi imperfecti (z.B. Colletotrichum lagenarium, Piricularia oryzae, Cercospora nicotinae), Ascomyceten (z.B. Venturia inaequalis);
Bakterien, wie z.B. Pseudomonaden (Pseudomonas lachrymans, Pseudomonas tomato, Pseudomonas tabaci); Xanthomonaden (z.B. Xanthomonas oryzae, Xanthomonas vesicatoria); Erwinia (z.B. Erwinia amylovora); und Viren, wie z.B. das Tabakmosaikvirus.

Die erfindungsgemässen Verbindungen können zum Schutz von Pflanzen aus unterschiedlichen Nutzkulturen eingesetzt werden.

Für den Einsatz der erfindungsgemässen Verbindungen der Formel I im Rahmen der Erfindung sind beispielsweise folgende Pflanzenarten geeignet:
Getreide (Weizen, Gerste, Roggen, Hafer, Reis, Sorghum und Verwandte); Rüben (Zucker- und Futterrüben); Kern-, Stein- und Beerenobst (Aepfel, Birnen, Pflaumen, Pfirsiche, Mandeln, Kirschen, Erdbeeren, Himbeeren und Brombeeren); Hülsenfrüchte (Bohnen, Linsen, Erbsen, Soja); Oelkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Grapefruit, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Blumen, Sträucher, Laubbäume und Nadelbäume wie Koniferen). Diese Aufzählung stellt keine Limitierung dar.

Als besonders geeignete Zielkulturen für die Anwendung des erfindungsgemässen Verfahrens sind folgende Pflanzen anzusehen: Gurke, Tabak, Reben, Reis, Pfeffer, Kartoffeln, Tomate, Weizen, Gerste, Birne und Apfel.

Die Verbindungen der Formel I werden aus 2,6-Dihalogen-isonicotinsäurehalogeniden, -anhydriden oder -azoliden gewonnen.

Die Verbindungen der Formel I werden hergestellt, indem man umsetzt:

a) ein Isonicotinsäurehalogenid der Formel II

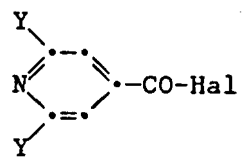

(II)

oder

b) ein Isonicotinsäureanhydrid der Formel IV

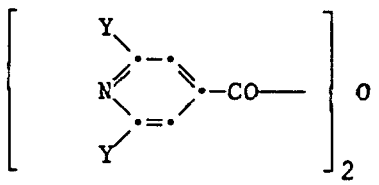

(IV)

oder

c) ein Isonicotinsäureazolid der Formel V

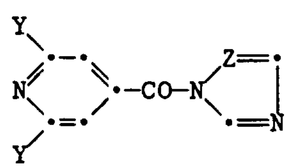

(V)

oder

d) ein Isonicotinsäurederivat der Formel VI

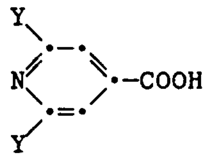

(VI)

mit einem Alkohol der Formel III

A-Q-XH (III);

wobei Z CH oder N darstellt und Y, A, Q und X die unter Formel I angegebenen Bedeutungen besitzen.

Die Umsetzungen werden vorteilhafterweise in Gegenwart eines inerten Lösungsmittels durchgeführt.

Die Reaktionvarianten (a) und (b) verlaufen in Gegenwart einer Base.

Als geeignete Temperaturen gelten für die Reaktionsvarianten (a), (b) und (c) -20° bis 150°C, vorzugsweise 0 bis 80°C und für die Reaktion (d) 0° bis 180°C, vorzugsweise 10° bis 110°C.

Für die Reaktionsvariante (d) wird ein Katalysator benötigt. Als solcher kommen in Betracht Lewissäuren, wie z.B. Bortrifluoriddiethyletherat oder Mineralsäuren, wie z.B. Schwefelsäure, HCl oder HBr (gasförmig), aber auch Basen wie z.B. tertiäre Amine, Pyridine oder alkalische Alkoholate. Weitere Beispiele für geeignete Katalysatoren sind dem Fachmann geläufig.

Als Basen zur Säurebindung in den Verfahrensvarianten (a) und (b) kommen organische und anorganische Basen in Betracht, z.B. tertiäre Amine wie Trialkylamine (Trimethylamin, Triethylamin, Tripropylamin usw.), Pyridin, Pyridinbasen (4-Dimethylaminopyridin, 4-Pyrrolidylaminopyridin usw.), und Oxide und Hydroxide, Carbonate und Hydrogencarbonate von Alkali- und Erdalkalimetallen sowie Alkaliacetate.

In den Verfahrensvarianten (a) und (b) werden als Reaktionsmedien in Anpassung an die jeweiligen Reaktionsbedingungen geeignete reaktionsinerte Lösungs- und Verdünnungsmittel verwendet. Als Beispiele sind zu nennen: aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon; sowie Gemische solcher Lösungsmittel untereinander.

Umsetzungsreaktionen analog den Verfahrensvarianten (a) bis (d) sind in der Literatur beschrieben, z.B. in Acta. Fac. Pharm. Bohemoslovenica IV, 1962,65.

Die Ausgangsstoffe zur Herstellung der Verbindungen der Formel I sind bekannt oder lassen sich nach bekannten Methoden herstellen. So ist die Herstellung von Alkoholen der Formel III z.B. beschrieben in Houben-Weyl, Bd. 6/1a/1b und Bd. 9.

Die Herstellung der als Ausgangsstoffe verwendeten Verbindungen der Formel V

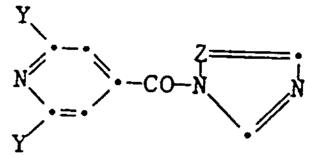

(V)

wird durch Umsetzung von Verbindungen der Formel II

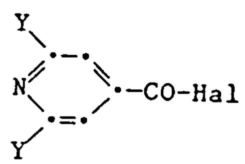

(II)

mit einem Azol der Formel VII

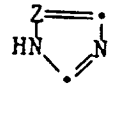

(VII)

in Gegenwart einer Base in einem inerten Lösungsmittel durchgeführt, wobei Y die unter Formel I angegebenen Bedeutungen hat, Hal für Halogen, vorzugsweise für Chlor, steht und Z ein N-Atom oder CH

darstellt.

Die Reaktionstemperaturen für die vorhergehend beschriebene Synthese betragen -50° bis 200°C, bevorzugt 10° bis 100°C.

Als Basen und Lösungsmittel kommen die für die Herstellung der Verbindungen der Formel I angegebenen in Frage.

Umsetzungen von Säurehalogeniden mit Azolen sind in Angew. Chemie 1962, S. 409-411 beschrieben.

Die Ausgangsstoffe der Formel V

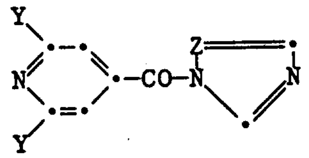

(V)

worin Y gleichzeitig Fluor, Chlor, Brom oder Jod bedeutet und Z entweder N oder CH darstellt, sind wertvolle Zwischenprodukte zur Herstellung der erfindungsgemässen Verbindungen der Formel I. Bei den Verbindungen der Formel V handelt es sich um neue Stoffe, die ebenfalls eine gegen die erwähnten Phytopathogene schützende Aktivität aufweisen. Sie stellen einen Bestandteil der vorliegenden Erfindung dar.

Die im Rahmen der Erfindung zur Anwendung gelangenden Mittel zum Schutz von Pflanzen gegen Krankheiten, welche die Verbindungen der Formel I als Aktivstoffe enthalten, sind als Teil der Erfindung zu betrachten.

Wirkstoffe der Formel I werden üblicherweise in Form von Zusammensetzungen verwendet und können gleichzeitig oder nacheinander mit weiteren Wirkstoffen auf die Pflanze oder deren Umgebung gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein Verfahren zur Anwendung eines Wirkstoffes der Formel I bzw. eines agrochemischen Mittels, das mindestens einen dieser Wirkstoffe enthält, ist das Aufbringen auf die Pflanze (Blattapplikation). Die Wirkstoffe der Formel I können aber auch über den Erdboden durch das Wurzelwerk in die Pflanze gelangen (Bodenapplikation), indem man den Standort der Pflanze mit einer flüssigen Zubereitung tränkt oder die Substanzen in fester Form in den Boden einbringt z.B. in Form von Granulat. Die Verbindungen der Formel I können aber auch auf Samenkörner aufgebracht werden (Coating), indem man die Körner entweder in einer flüssigen Zubereitung des Wirkstoffs tränkt oder sie mit einer festen Zubereitung beschichtet (Beizapplikation). Darüber hinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmitteln eingesetzt. Zu diesem Zweck werden sie z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt bei 100 g bis 2 kg AS/ha, insbesondere bei 100 g bis 600 g AS/ha.

Die Formulierungen, d.h. die den Wirkstoff der Formel I und gegebenenfalls einen festen oder flüssigen Zusatzstoff enthaltenden Mittel, Zubereitungen oder Zusammensetzungen werden hergestellt durch inniges Vermischen und/oder Vermahlen der Wirkstoffe mit Streckmitteln, wie z.B. mit Lösungsmitteln, festen Trägerstoffen, und gegebenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie

deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid; sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht-sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I nicht-ionogene, kation-und/oder anionaktive Tenside mit guten Emulgier-, Dispergier- und Netzeigenschaften in Betracht. Unter Tensiden sind auch Tensidgemische zu verstehen.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedere, gegebenenfalls halogenierte Alkyl-, Benzyl- oder niedere Hydroxyalkylreste aufweisen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure oder von natürlichen Fettsäuregemischen, die z.B. aus Kokosnuss- oder Talgöl gewonnen werden können, genannt.

Als synthetische Tenside können insbesondere Fettalkoholsulfonate, Fettalkoholsulfate, sulfonierte Benzimidazolderivate oder Alkylarylsulfonate Verwendung finden. Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die agrochemischen Zubereitungen enthalten in der Regel 0,1 bis 99 Gew.-%, insbesondere 0,1 bis 95 Gew.-%, Wirkstoff der Formel I, 99,9 bis 1 Gew.-%, insbesondere 99,8 bis 5 Gew.-%, eines festen oder flüssigen Zusatzstoffes und 0 bis 25 Gew.-%, insbesondere 0,1 bis 25 Gew.-%, eines Tensides.

Die nachfolgenden Beispiele dienen zur näheren Erläuterung der Erfindung, ohne dieselbe einzuschränken.

## 1. Herstellungsbeispiele

Beispiel 1.1: Herstellung von 1,2,4-Triazol-1'yl-methyloxycarbonyl-4'yl-(2,6-dichlorpyridin)

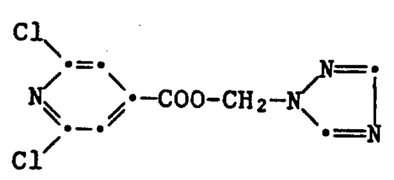

Zu 3.3 g Hydroxymethyl-1,2,4-triazol-1'yl in 50 ml absolutem Pyridin werden unter Eiskühlung bei 10 - 15° 7,8 g 2,6-Dichlorisonicotinsäurechlorid zugetropft. Anschliessend wird während 4 Stunden bei Raumtemperatur gerührt, abgekühlt und zwischen Wasser und Dichlormethan verteilt. Der organische Extrakt wird mit Wasser gewaschen, getrocknet und eingedampft. Nach Trocknen am Hochvacuum und anschliessendem Umkristallisieren aus Tetrahydrofuran/Ligroin verbleiben 5.3 g der Titelverbindung vom Smp. 78-80° C.

Beispiel 1.2: Herstellung von 2,6-Dichlorisonicotinsäure-benzylester

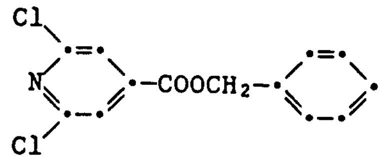

7.8 g 2,6-Dichlorisonicotinsäurechlorid, gelöst in 10 ml Acetonitril, werden tropfenweise zu einer durch Kühlen bei 15 - 20 ° C gehaltenen Lösung von 5,4 g Benzylalkohol, 0,5 g 4-Dimethylaminopyridin und 3,0 g Pyridin in 50 ml Acetonitril gegeben. Nach Rühren über Nacht bei Raumtemperatur wird auf Eiswasser gegossen, in Methylenchlorid aufgenommen, mit Wasser gewaschen, getrocknet und eingedampft. Das verbleibende Oel kristallisiert aus Pentan. Es resultieren weisse Kristalle vom Smp. 39-41 ° C.

Beispiel 1.3: 2,6-Dichlor-isonicotinsäure-α-(4-chlorphenyl)-benzylester

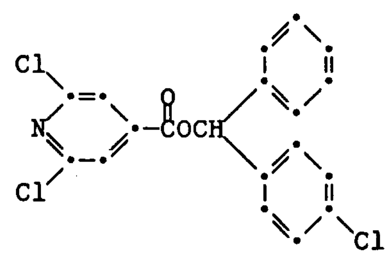

3.28 g (0,015 Mol) α-(4-Chlorphenyl)-benzylalkohol werden in 30 ml Tetrahydrofuran mit 0,5 ml Triethylamin gelöst. Nach der Zugabe von 4,12 g (0,017 Mol) 2,6-Dichlorisonicotinoylimidazol wird bei Raumtemperatur gerührt. Die Suspension geht dabei innerhalb von 30 Minuten in Lösung. Die Umsetzung ist nach 3,5 Stunden beendet. Im Dünnschichtchromatogramm sind dann nur noch Spuren des eingesetzten Alkohols nachweisbar. Man extrahiert mit Wasser und Essigsäureethylester, trocknet die organische Phase über Natriumsulfat und erhält nach der Entfernung des Lösungsmittels am Rotationsdampfer 6,1 g Oel. Dieses wird über Kieselgel mit einem Gemisch aus 4 Teilen Hexan und 1 Teil Essigsäureethylester chromatographiert. Aus den Reinfraktionen erhält man nach der Entfernung des Lösungsmittels 5,45 g in Form eines farblosen, zähen Harzes.

$^1$H-NMR ($CDCl_3$; ppm-Werte): 7,1 ( >CHO-); 7,3-7,4 (9 arom. H); 7,8 (2H; Pyridinring).

Beispiel 1.4: Herstellung des Vorproduktes zu Beispiel 1.3 α-(4-Chlorphenyl)-benzylalkohol

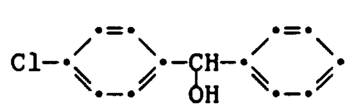

15,2 g (0,07 Mol) Phenyl-(4-chlorphenyl)-keton werden in 30 ml Tetrahydrofuran und 10 ml Methanol gelöst. Man fügt unter Rühren allmählich 1,32 g (0,035 Mol) Natriumborhydrid zu, und hält die exotherme Reaktion durch Kühlung zwischen 25 und 30 ° C. Nach einer Stunde lässt sich mittels Dünnschichtchromatographie kein Keton mehr nachweisen. Das Gemisch wird dann mit Wasser und Chloroform extrahiert, das Chloroform mit Natriumsulfat getrocknet und am Rotationsverdampfer entfernt. Die Rohausbeute beträgt 16,2 g (Oel). Davon werden 15,5 g bei 220 ° C und 30 mbar im Kugelrohrofen destilliert. Man erhält 14,3 g Oel das gaschromatographisch zu über 95 % rein ist und beim Erkalten kristallin erstarrt. 13,3 g werden aus einem Gemisch aus 25 ml n-Hexan und 5 ml Cyclohexan umkristallisiert. Die Ausbeute beträgt 12,2 g; Smp. 58-60 ° C.

Gemäss den beschriebenen Herstellungsweisen werden die nachfolgend aufgeführten Verbindungen erhalten.

In den Tabellen (z.B. Tabelle 3) haben die Reste $A_2$ bis $A_{10}$ folgende Bedeutungen:

$A_2$ = 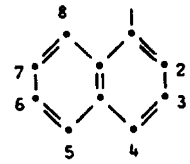

$A_3$ = 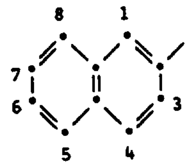

$A_4$ = 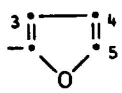

$A_5$ = 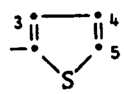

$A_6$ = 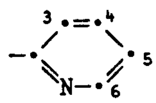

$A_7$ = 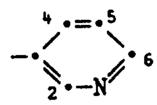

$A_8$ = 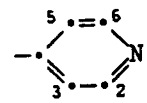

$A_9$ = 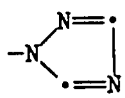

$A_{10}$ = 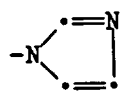

Tabelle 1

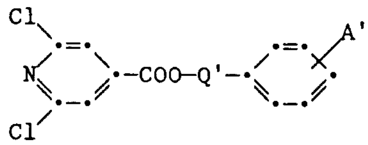

| Nr. | Q' | A' | physik. Daten |
|---|---|---|---|
| 1.1 | $-CH_2$ | H | Smp. 39-41° |
| 1.2 | $-CH(CH_3)-$ | H | |
| 1.3 | $-CH(C_2H_5)-$ | H | |
| 1.4 | -CH(- 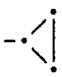 )- | H | |
| 1.5 | $-CH(CH_3)-$ | 2,4-di-Cl | $n_D^{50}$ 1,5731 |
| 1.6 | $-CH(C_2H_5)-$ | 2,4-di-Cl | $n_D^{50}$ 1,5683 |
| 1.7 | -CH(Phenyl)- | 4-Cl | Harz |
| 1.8 | -CH(4-Chlorphenyl)- | 3,4-di-$OCH_3$ | $n_D^{50}$ 1,5939 |
| 1.9 | $-CH_2-$ | 2-Cl | |
| 1.10 | $-CH_2-$ | 3-Cl | |
| 1.11 | $-CH_2-$ | 4-$CH_3$ | |
| 1.12 | $-CH_2-$ | 4-$NO_2$ | |
| 1.13 | $-CH(C_2H_5)-$ | 4-$OCH_3$ | $n_D^{30}$ 1,5604 |
| 1.14 | $-CH(C_2H_5)-$ | H | |
| 1.15 | $-CH(C_2H_5)-$ | 4-Cl | |
| 1.16 | $-CH_2$ | 3-F | |
| 1.17 | $-CH_2-$ | 4-$CF_3$ | Smp. 81-83°C |
| 1.18 | $-CH_2-$ | 2-F | |
| 1.19 | $-CH_2-$ | 4-F | |
| 1.20 | $-CH_2-CH_2-$ | H | |
| 1.21 | $-CH_2-$ | 2-$NO_2$ | |
| 1.22 | $-CH_2-$ | 2-$CH_3$ | |
| 1.23 | $-CH_2-$ | 4-$C_6H_5$ | |
| 1.24 | $-CH_2-$ | 4-O-$C_6H_5$ | |
| 1.25 | $-C(Phenyl)_2-$ | H | |
| 1.26 | $-CH(COOCH_3)-$ | H | $n_D^{30}$ 1,5593 |

Tabelle 1 (Fortsetzung)

| Nr. | Q' | A' | physik. Daten |
|---|---|---|---|
| 1.27 | $-CH(COOC_2H_5)-$ | H | |
| 1.28 | $-CH(COOC_3H_7(i))$ | H | |
| 1.29 | $-CH(COOC_4H_9(n)$ | H | |
| 1.30 | $-CH(COOCH_3)-$ | 2,4-di-Cl | |
| 1.31 | $-CH(COOCH_3)-$ | 2-Cl | |
| 1.32 | $-CH(COOCH_3)-$ | 3-Cl | |
| 1.33 | $-CH(COOCH_3)-$ | 4-Cl | |
| 1.34 | $-CH(COOCH_3)-$ | 2-F | |
| 1.35 | $-CH_2-$ | 3-CN | |
| 1.36 | $-C(CH_3)COOCH_3$ | H | |
| 1.37 | $-CH_2-CH=CH-$ | H | $n_D^{30}$ 1,5973 |
| 1.38 | $-CH_2-CH_2-CH_2-$ | 3,4-di-$OCH_3$ | |
| 1.39 | $-C(CH_3)_2$ | H | |
| 1.40 | -CH(4-chlorphenyl)- | 2,4-di-$OCH_3$ | |
| 1.41 | $-CH(C_6H_5)-$ | H | Oel |
| 1.42 | $-CH(CH_3)-$ | 4-Br | |
| 1.43 | $-CH(C_6H_5)-$ | 4-Br | |
| 1.44 | $-CH(C_6H_5)-$ | 4-Cl | |
| 1.45 | $-CH(CH_3)-$ | 4-$C_6H_5$ | |
| 1.46 | -CH(4-Methoxyphenyl)- | 4-$OCH_3$ | |
| 1.47 | $-CH(C_6H_5)CH_2CH_2-$ | H | |
| 1.48 | $-CH(CH_3)CH_2CH_2-$ | H | |
| 1.49 | $-CHCOC_6H_5$ | H | Smp. 165-167°C |
| 1.50 | $-CH(CH_3)-$ | 2,4,6-Trimethyl | |
| 1.51 | $-CH(CH_2)_2CH_3-$ | H | |
| 1.52 | $-CH(CH_3)-$ | 2-Cl | |
| 1.53 | $-CH(CH_3)-$ | 4-Cl | |
| 1.54 | $-CH(CH_3)-$ | 3,4-di-$OCH_3$ | $n_D^{30}$ 1,5634 |
| 1.55 | $-CH_2-$ | 2,4-di-F | |
| 1.56 | $-CH(CH_3)-$ | 2,4-dimethyl | |
| 1.57 | $-CH(CH_3)-$ | 2-F | |
| 1.58 | $-CH(CH_3)-$ | 4-F | |
| 1.59 | $-CH(CH(CH_3)_2)-$ | H | |

Tabelle 1 (Fortsetzung)

| Nr. | Q' | A' | physik. Daten |
|---|---|---|---|
| 1.60 | $-CH(CH_3)-$ | $2-OCH_3$ | |
| 1.61 | $-CH(CH_3)-$ | $3-OCH_3$ | |
| 1.62 | $-CH(CH_3)-$ | $4-OCH_3$ | Smp. 46-48°C |
| 1.63 | $-CH(4-CH_3OC_6H_4)-$ | H | |
| 1.64 | $CH(C_3H_7(n))-$ | $4-OCH_3$ | |
| 1.65 | $-CH(CH_3)-$ | $O-CH_3$ | |
| 1.66 | $-CH(CH_3)-$ | $3-CH_3$ | $n_D^{30}$ 1,5594 |
| 1.67 | $-CH(CH_3)-$ | $4-CH_3$ | |
| 1.68 | $-CH(CH(CH_3)C_2H_5)-$ | H | |
| 1.69 | $-CH(CH_3)-$ | $2-NO_2$ | |
| 1.70 | $-CH(CH_3)-$ | $3-NO_2$ | |
| 1.71 | $-CH(CH_3)-$ | $4-NO_2$ | |
| 1.72 | $-CH(CCl_3)-$ | H | |
| 1.73 | $-CH(CF_3)-$ | H | $n_D^{30}$ 1,5259 |
| 1.74 | $-CH(CH_3)-$ | $2-CF_3$ | |
| 1.75 | $-CH(CH_3)-$ | $4-CF_3$ | |
| 1.76 | $-CH(CH_3)-$ | 2,3,4-Trimethoxy | |
| 1.77 | $-CH(CH_3)-$ | 3,4,5-Trimethoxy | |
| 1.78 | $-CH_2-$ | 2,6-di-Cl | |
| 1.79 | $-CH_2-$ | 2,4-di-Cl | |
| 1.80 | $-C(CH_3)_2-$ | $4-C_6H_5$ | |
| 1.81 | -CH[CO-(2,4-di-Cl-Phenyl)] | 2,4-di-Cl | |
| 1.82 | -CH[CO-(4-OMe-Phenyl)] | $4-OCH_3$ | |
| 1.83 | -CH(- 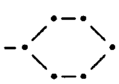 )- | H | |
| 1.84 | $-C(CH_3)_2CH_2-$ | H | Smp. 86-88°C |
| 1.85 | $-CH_2-$ | 2,3-di-Cl | Smp. 78-80°C |
| 1.86 | $-CH_2CH(n-C_3H_7)-$ | 2,4-di-Cl | Smp. 91-93°C |

Tabelle 1 (Fortsetzung)

| Nr. | Q' | A' | physik. Daten |
|---|---|---|---|
| 1.87 | $-CH(CH_3)-$ | $2-CH_3-4-$(O— 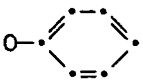 —Cl) | Smp. 79-81°C |
| 1.88 | $-CH(CN)-$ | H | $n_D^{30}$ 1,5673 |
| 1.89 | $-CH(CH_3)CH_2-$ | $2-OCH_3$ | $n_D^{30}$ 1,5563 |
| 1.90 | $-CH(CH_3)-$ | $2-OCHF_2-4-F-5-NO_2$ | $n_D^{30}$ 1,5493 |

Tabelle 2

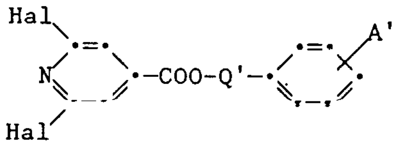

| Nr. | Q' | Hal | A' | physik. Daten |
|---|---|---|---|---|
| 2.1 | $-CH_2-$ | Br | H | Smp. 89-91°C |
| 2.2 | $-CH_2-$ | F | H | |
| 2.3 | $-CH_2-$ | J | H | |
| 2.4 | $-CH(CH_3)-$ | Br | 2,4-di-Cl | |
| 2.5 | $-CH(C_2H_5)-$ | Br | 2,4-di-Cl | |
| 2.6 | $-CH(CH_3)-$ | J | H | |
| 2.7 | -CH-(4-Chlorphenyl)- | Br | 3,4-di-$OCH_3$ | |
| 2.8 | $-CH_2-$ | | 4-Me | |
| 2.9 | $-CH(C_6H_5)-$ | Br | H | |
| 2.10 | $-CH(C_6H_5)-$ | J | H | |
| 2.11 | $-C(C_6H_5)_2-$ | Br | H | |
| 2.12 | $-CH_2-$ | Br | 4-$C_6H_5$ | |
| 2.13 | $-CH_2-$ | Br | 4-$OC_6H_5$ | |
| 2.14 | $-CH_2-$ | F | 3-$NO_2$ | |
| 2.15 | $-CH_2-CH_2$ | Br | H | |
| 2.16 | $-CH_2-CH_2-CH_2-$ | Br | H | |
| 2.17 | $-CH_2-CH_2-$ | J | H | |
| 2.18 | -CH( 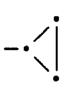 )- | Br | 2,4-di-Cl | |
| 2.19 | $-CH(COOCH_3)-$ | Br | 2,4-di-Cl | |
| 2.20 | $-CH(C_6H_5)-$ | Br | 4-Cl | |
| 2.21 | $-C(CH_3)_2$ | Br | H | |
| 2.22 | -CH( 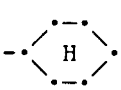 )- | Br | H | |
| 2.23 | -CH(CO-Phenyl)- | Br | H | |
| 2.24 | $-CH_2-$ | Br | 4-Cl | Smp. 91-92°C |

Tabelle 3

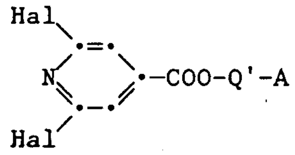

| Nr. | Q' | Hal | A | physik. Daten |
|---|---|---|---|---|
| 3.1 | $-CH_2-$ | Cl | $A_2$ | Smp. 84-86°C |
| 3.2 | $-CH_2-$ | Cl | $A_3$ | Smp. 86-90°C |
| 3.3 | $-CH_2-$ | Cl | $A_4$ | Smp. 39-41°C |
| 3.4 | $-CH_2-$ | Cl | $A_5$ | Smp. 42-43°C |
| 3.5 | $-CH_2-$ | Cl | $A_6$ | Smp. 53-54°C |
| 3.6 | $-CH_2-$ | Cl | $A_7$ | Smp. 58-59°C |
| 3.7 | $-CH_2-$ | Cl | $A_8$ | Smp. 62-64°C |
| 3.8 | $-CH_2-$ | Cl | $A_9$ | Smp. 78-80°C |
| 3.9 | $-CH_2-$ | Cl | $A_{10}$ | Smp. 81-84°C |
| 3.10 | $-CH_2-$ | Br | $A_2$ | |
| 3.11 | $-CH_2-$ | Br | $A_3$ | |
| 3.12 | $-CH_2-$ | Br | $A_4$ | |
| 3.13 | $-CH_2-$ | Br | $A_5$ | |
| 3.14 | $-CH_2-$ | Br | $A_6$ | |
| 3.15 | $-CH_2-$ | Br | $A_9$ | |
| 3.16 | $-CH_2-$ | Br | $A_{10}$ | |
| 3.17 | $-CH_2-$ | F | $A_2$ | |
| 3.18 | $-CH_2-$ | F | $A_3$ | |
| 3.19 | $-CH_2-$ | J | $A_4$ | |
| 3.20 | $-CH_2-$ | F | $A_6$ | |
| 3.21 | $-CH_2-$ | F | $A_9$ | |
| 3.22 | $-CH_2-CH_2-$ | Cl | $A_2$ | |
| 3.23 | $-CH_2-CH_2-$ | Cl | $A_9$ | |
| 3.24 | $-CH_2-CH_2-CH_2-$ | Cl | $A_3$ | |
| 3.25 | $-CH-(CH_3)-$ | Cl | $A_8$ | |
| 3.26 | $-CH(COOCH_3)-$ | Cl | $A_6$ | |
| 3.27 | $-CH(COOC_2H_5)-$ | Cl | $A_7$ | |
| 3.28 | $-CH(COOCH_3)-$ | Cl | $A_9$ | |

Tabelle 3: (Fortsetzung)

| Nr. | Q' | Hal | A | physik. Daten |
|---|---|---|---|---|
| 3.29 | $-CH(COO(C_3H_7)i)-$ | Br | $A_{10}$ | |
| 3.30 | $-CH_2-CH_2-$ | Br | $A_6$ | |
| 3.31 | $-CH_2-CH_2-CH_2-$ | J | $A_9$ | |
| 3.32 | $-C(CH_3)_2-$ | Cl | $A_3$ | |
| 3.33 | $-C(CH_3)_2-$ | Cl | $A_4$ | |
| 3.34 | $-C(CH_3)_2-$ | Cl | $A_9$ | |
| 3.35 | $-C(CH_3)_2-$ | Cl | $A_9$ | |
| 3.36 | $-C(CH_3)_2-$ | Cl | $A_{10}$ | |
| 3.37 | $-C(CH_3)_2-$ | Br | $A_7$ | |
| 3.38 | $-CH(CH_3)-$ | Cl | $A_2$ | |
| 3.39 | $-CH(C_6H_5)-$ | Cl | $A_8$ | |
| 3.40 | $-CH(C_2H_5)-$ | Cl | $A_9$ | |
| 3.41 | $-CH_2(C(CH_3)_2)-$ | Cl | $A_{10}$ | |
| 3.42 | $-CH(2,4\text{-di-Cl-}C_6H_3)-$ | Br | $A_4$ | |
| 3.43 | $-CH(2,4\text{-di-Cl-}C_6H_3)-$ | Cl | $A_7$ | |
| 3.44 | $-CH(2,4\text{-di-Cl-}C_6H_3)-$ | Cl | $A_8$ | |
| 3.45 | $-CH_2-CH_2-CH_2-$ | Cl | $A_9$ | |
| 3.46 | $-CH_2-CH(CH_3)-$ | Cl | $A_9$ | |
| 3.47 | $-CH_2CH(CH_3)-[A]$ | Cl | $A_{10}$ | |
| 3.48 | $-CH_2-CH(C_2H_5)-[A]$ | Cl | $A_9$ | |
| 3.49 | $-CH_2-CH(C_3H_7i)-[A]$ | Cl | $A_9$ | |
| 3.50 | $-CH_2-C(C_2H_5)_2-[A]$ | Cl | $A_9$ | |
| 3.51 | $-CH_2-CH(C_4H_9\text{-n})-[A]$ | Cl | $A_9$ | |
| 3.52 | -CH[CO-(2,4-di-Cl-Phenyl)] | Cl | $A_7$ | |

Tabelle 4:

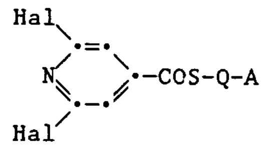

| Nr. | Q | Hal | A | physik. Daten |
|---|---|---|---|---|
| 4.01 | $-CH_2-$ | Cl | Phenyl | Smp. 63-65°C |
| 4.02 | $-CH_2-$ | Br | Phenyl | |
| 4.03 | $-CH_2-$ | Cl | $4-Cl-C_6H_4-$ | |
| 4.04 | $-CH_2-$ | Cl | $2,4-DiCl-C_6H_3-$ | |
| 4.05 | $-CH-CH_3-$ | Cl | $2,4-DiCl-C_6H_3-$ | |
| 4.06 | $-CH(COOCH_3)-$ | Br | $2,4-DiCl-C_6H_3-$ | |
| 4.07 | $-CH_2-$ | F | Phenyl | |
| 4.08 | $-CH_2CH_2$ | Cl | Phenyl | |
| 4.09 | $-CH_2CH_2CH_2-$ | Cl | Phenyl | |
| 4.10 | -CH(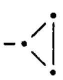)- | Cl | Phenyl | |
| 4.11 | $-CH_2-$ | Cl | $A_2$ | |
| 4.12 | $-CH_2-$ | Br | $A_4$ | |
| 4.13 | $-CH_2-$ | Cl | $A_6$ | |
| 4.14 | $-CH_2-$ | J | $A_7$ | |
| 4.15 | $-CH_2-$ | Cl | $A_8$ | |
| 4.16 | $-CH(CH_3)-$ | Cl | $2,4-di-Cl-C_6H_3-$ | |
| 4.17 | $-CH_2-$ | Cl | $C_6H_5-C_6H_4-$ | |
| 4.18 | $-CH-(CH_3)-$ | Cl | diphenyl(1',4')- | |
| 4.19 | $-CH_2-$ | Cl | $A_4$ | Smp. 88-90°C |

2. Formulierungsbeispiele für flüssige Wirkstoffe der Formel I (% = Gewichtsprozent)

| 2.1 Emulsions-Konzentrate | a) | b) | c) |
|---|---|---|---|
| Wirkstoff aus den Tabellen | 25 % | 40 % | 50 % |
| Ca-Dodecylbenzolsulfonat | 5 % | 8 | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2.2 Lösungen | a) | b) | c) | d) |
|---|---|---|---|---|
| Wirkstoff aus den Tabellen | 80 % | 10 % | 5 % | 95 % |
| Ethylenglykolmonomethylether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190 °C) | - | - | 94 % | - |
| (MG = Molekulargewicht) | | | | |

Die Lösungen sind zur Anwendung in Form kleinster Tropfen geeignet.

| 2.3 Granulate | a) | b) |
|---|---|---|
| Wirkstoff aus den Tabellen | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4 Stäubemittel | a) | b) |
|---|---|---|
| Wirkstoff aus den Tabellen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 2.5 Spritzpulver | a) | b) | c) |
|---|---|---|---|
| Wirkstoff aus den Tabellen | 25 % | 50 % | 75 % |
| Na-Ligninsulfonat | 5 % | 5 % | |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle homogen vermahlen. Man erhält Spritzpulver, die sich mit Wasser zu Suspensionen jeder gewünschten Konzentration verdünnen lassen.

| 2.6 Emulsions-Konzentrat | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 2.7 Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 2.8 Extruder Granulat | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.9 Umhüllungs-Granulat | |
|---|---|
| Wirkstoff aus den Tabellen | 3 % |
| Polyethylenglykol (MG 200) | 3 % |
| Kaolin | 94 % |
| (MG = Molekulargewicht) | |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| 2.10 Suspensions-Konzentrat | |
|---|---|
| Wirkstoff aus den Tabellen | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält ein Suspensions-Konzentrat, aus welchem durch Verdünnen mit Wasser Suspensionen jeder gewünschten Konzentration hergestellt werden können.

## 3. Biologische Beispiele

### Beispiel 3.1: Schutz gegen Colletotrichum lagenarium auf Cucumis sativus L.

a) Gurkenpflanzen werden nach 2-wöchiger Anzucht mit einer aus Spritzpulver des Wirkstoffes hergestellten Spritzbrühe besprüht (Konzentration: 20 ppm).

Nach 3 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23°C inkubiert. Die Inkubation wird dann bei normaler Luftfeuchigkeit und bei 22° bis 23°C weitergeführt.

Die Beurteilung der Schutzwirkung erfolgt aufgrund des Pilzbefalls 7-8 Tage nach der Infektion.

Unbehandelte aber infizierte Kontrollpflanzen wiesen in dem Test einen Pilzbefall von 100 % auf.

Verbindungen aus den Tabellen 1 bis 4 bewirkten einen guten Schutz gegen Colletotrichum lagenarium. So blieben Pflanzen, die z.B. mit den Verbindungen Nr. 1.1, 1.5. 1.6, 1.7, 1.8, 1.13, 1.17, 1.26, 1.37, 1.41, 1.54, 1.62, 1.66, 1.73, 1.84, 1.87, 1.88, 1.89, 2.1 und 2.24 behandelt wurden, fast völlig frei von Colletotrichum (Befall 10 bis 0 %).

b) Gurkensamen werden mit einer Lösung des Wirkstoffes gebeizt (Konzentration: 180 g/100 kg Samen). Die Samen werden ausgesät. Nach 4 Wochen werden die Pflanzen mit einer Sporensuspension $1.5 \cdot 10^5$ Sporen/ml) des Pilzes infiziert und für 36 Stunden bei hoher Luftfeuchtigkeit und einer Temperatur von 23°C inkubiert. Die Inkubation wurde dann bei normaler Luftfeuchtigkeit und bei 22° bis 23°C weitergeführt. Die Beurteilung der Schutzwirkung erfolgt aufgrund des Pilzbefalls 7-8 Tage nach der Infektion.

Infizierte Kontrollpflanzen, deren Samen nicht behandelt wurden, wiesen in dem Test einen Pilzbefall von 100 % auf.

### Beispiel 3.2: Wirkung gegen Xanthomonas oryzae auf Reis (Oryza sativa)

a) Reispflanzen der Sorte "Calora" oder "S6" werden nach 3-wöchiger Anzucht im Gewächshaus mit der Prüfsubstanz in Form einer Spritzbrühe (0,02 % Aktivsubstanz) besprüht. Nach eintägigem Antrocken dieses Spritzbelags werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der residual Wirksamkeit der Prüfsubstanz.

b) Reispflanzen der Sorte "Calora" oder "S6" werden nach 3-wöchiger Anzucht im Gewächshaus mit einer Suspension der Prüfsubstanz begossen (0,006 % Aktivsubstanz bezogen auf das Erdvolumen). Drei Tage nach dieser Behandlung werden die Pflanzen in einem Klimaraum bei 24°C und 75-85 % relativer Luftfeuchtigkeit aufgestellt und infiziert. Die Infektion erfolgt, indem die Blattspitzen mit einer Schere, die zuvor in eine Suspension von Xanthomonas oryzae eingetaucht worden war, abgeschnitten werden. Nach 10-tägiger Inkubation werden die angeschnittenen Blätter bei Befall welk, rollen sich ein und werden nekrotisch. Das Ausmass dieser Krankheitssymptome dient zur Beurteilung der systemischen Wirksamkeit der Prüfsubstanz.

Verbindungen aus den Tabellen 1 bis 4 zeigten eine gute Schutzwirkung gegen Xanthomonas oryzae. So reduzierten z.B. im Test (a) die Verbindungen 1.7, 1.8 und 3.8 und im Test (b) die Verbindungen 1.6 und 1.8 den Bakterienbefall auf 0 bis 20 %. Unbehandelte jedoch infizierte Kontrollpflanzen wiesen dagegen einen Krankheitsbefall von 100 % auf.

### Beispiel 3.3: Wirkung gegen Xanthomonas vesicatoria auf Paprika (Capsicum annuum)

a) Paprikapflanzen der Sorte "California Wonder" werden nach 3-wöchiger Anzucht im Gewächshaus mit der Prüfsubstanz in Form einer Spritzbrühe (0,02 % Aktivsubstanz) besprüht. Nach eintägigem Antrocknen dieses Spritzbelages werden die Pflanzen in einem Klimaraum bei 26°C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der residualen Wirksamkeit der Prüfsubstanz.

b) Paprikapflanzen der Sorte "California Wonder" werden nach 3-wöchiger Anzucht im Gewächshaus mit einer Suspension der Prüfsubstanz begossen (0,006 % Aktivsubstanz bezogen auf das Erdvolumen).

Drei Tage nach dieser Behandlung werden die Pflanzen in einem Klimaraum bei 26 °C und 95-100 % relativer Luftfeuchtigkeit aufgestellt und durch Besprühen der Blattunterseiten mit einer standardisierten Suspension von Xanthomonas vesicatoria infiziert. Nach 6-tägiger Inkubation bilden sich bei Befall auf den Blättern runde, anfangs wässrige, später nekrotische, aufgehellte Flecken. Das Ausmass dieser Flecken dient zur Beurteilung der systemischen Wirksamkeit der Prüfsubstanz.

Verbindungen aus den Tabellen 1 bis 4 zeigten eine gute Schutzwirkung gegen Xanthomonas vesicatoria. So reduzierten z.B. im Test (a) die Verbindungen 1.7, 1.8, 3.8 und im Test (b) 1.7 und 3.8 den Bakterienbefall 0 bis 20 %. Unbehandelte jedoch infizierte Kontrollpflanzen wiesen dagegen einen Krankheitsbefall von 100 % auf.

Beispiel 3.4: Wirkung gegen Pseudomonas lachrymans auf Cucumis sativus L.

Gurkenpflanzen werden nach 2-wöchiger Anzucht mit einer aus Spritzpulver des Wirkstoffes hergestellten Spritzbrühe besprüht (Konzentration: 20 ppm).

Nach 1 Woche werden die Pflanzen mit einer Bakteriensuspension ($10^8$ Bakterien/ml) infiziert und für 7 Tage bei hoher Luftfeuchtigkeit und einer Temperatur von 23 °C inkubiert.

Die Beurteilung der Schutzwirkung erfolgt aufgrund des Bakterienbefalls 7-8 Tage nach der Infektion.

Unbehandelte aber infizierte Kontrollpflanzen wiesen in dem Test einen Krankheitsbefall von 100 % auf.

Verbindungen aus den Tabellen 1 bis 4 bewirkten einen guten Schutz gegen Pseudomonas lachrymans. So blieben Pflanzen, die z.B. mit der Verbindung Nr. 1.7 oder 3.8 behandelt wurden, fast völlig frei von Pseudomonas (Befall 10 bis 0 %).

Beispiel 3.5: Wirkung gegen Phytophthora infestans auf Tomatenpflanzen

a) Tomatenpflanzen werden nach 3-wöchiger Anzucht mit einer aus Spritzpulver des Wirkstoffes, wie oben beschrieben hergestellten Spritzbrühe (0,02 % Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100 % Luftfeuchtigkeit und 20 °C.

b) Zu Tomatenpflanzen wurde nach 3-wöchiger Anzucht eine aus Spritzpulver des Wirkstoffes, wie oben beschrieben hergestellte Spritzbrühe gegossen (0,002 % Aktivsubstanz bezogen auf das Erdvolumen). Es wurde darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgt nach einer Inkubation der infizierten Pflanzen während 5 Tagen bei 90-100 % relativer Luftfeuchtigkeit und 20 °C.

Verbindungen aus den Tabellen 1 bis 4 zeigten eine gute Schutzwirkung gegen Phytophthora. So reduzierte sich bei Applikation der Verbindung 1.07, 1.13, 1.17, 1.26, 1.37, 1.54, 1.62, 1.66, 1.87, 1.88, 1.89, 1.90, 2.1, 2.4 oder 3.8 der Befall in den Beispielen (a) und (b) auf 0 - 20 %.

Unbehandelte jedoch infizierte Pflanzen waren 100%-ig verwelkt.

Beispiel 3.6: Wirkung gegen Peronospora tabacina auf Tabak

a) Tabakpflanzen (8 Wochen alt) werden mit einer formulierten Lösung des Wirkungsstoffes besprüht (Konzentration: 200 ppm). Vier Tage nach der Behandlung werden die Pflanzen mit einer Sporangiensuspension von Peronospora tabacina ($10^4$ Sporen/ml) inokuliert, 20 Stunden im Dunkeln bei 25 °C und hoher Luftfeuchtigkeit aufbewahrt und dann bei normaler Tag/Nacht Wechselfolge weiterinkubiert.

b) Tabakpflanzen (8 Wochen alt) werden mit einer formulierten Lösung des Wirkungsstoffes durch Bodenapplikation behandelt (Konzentration: 6 ppm). Nach 4 Tagen werden die Pflanzen mit einer Sporangiensuspension von Peronospora tabacina ($10^4$ Sporen/ml) inokuliert, 20 Stunden im Dunkeln bei 25 °C und hoher Luftfeuchtigkeit aufbewahrt und dann bei normaler Tag/Nacht Wechselfolge weiterinkubiert.

Die Beurteilung der Symptome in den Tests a) und b) erfolgt aufgrund der mit Pilz befallenen Blattoberfläche.

Verbindungen aus den Tabellen 1 bis 4 zeigten eine gute Schutzwirkung gegen Peronospora tabacina.

Unbehandelte jedoch infizierte Pflanzen zeigten einen Befall von 80 bis 100 %.

Beispiel 3.7: Wirkung gegen Erysiphe graminis auf Gerste

a) Ca. 8 cm hohe Gerstenpflanzen wurden mit einer aus Spritzpulver des Wirkstoffes hergestellten Spritzbrühe (0,02 % Aktivsubstanz) besprüht. Nach 3 - 4 Stunden wurden die behandelten Pflanzen mit Konidien des Pilzes bestäubt. Die infizierten Gerstenpfalnzen wurden in einem Gewächshaus bei ca. 22 °C aufgestellt und der Pilzbefall nach 10 Tagen beurteilt.

b) Zu ca. 8 cm hohen Gerstenpflanzen wurde eine aus Spritzpulver des Wirkstoffes hergestellte Spritzbrühe gegossen (0,006 % Atkivsubstanz bezogen auf das Erdvolumen). Es wurde dabei darauf geachtet, dass die Spritzbrühe nicht mit den oberirdischen Pflanzenteilen in Berühung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen aus den Tabellen 1 bis 4, z.B. die Verbindungen 1.07 und 3.8 reduzierten den Pilzbefall auf weniger als 20 %, während unbehandelte aber infizierte Kontrollpflanzen zu 100 % befallen waren.

Beispiel 3.8: Wirkung gegen Pyricularia oryzae auf Reispflanzen

a) Reispflanzen werden nach 2-wöchiger Anzucht mit einer aus Spritzpulver des Wirkstoffes hergestellten Spritzbrühe (0,002 % Aktivsubstanz) besprüht. Nach 48 Stunden werden die behandelten Pflanzen mit einer Konidiensuspension des Pilzes infiziert. Nach 5 Tagen Inkubation bei 95-100 % relativer Luftfeuchtigkeit und 24 °C wird der Pilzbefall beurteilt.

b) Zu 2-wöchigen in Töpfen verpflanzte Reispflanzen wird eine aus Spritzpulver des Wirkstoffes hergestellte Spritzbrühe gegossen (0,006 % Aktivsubstanz bezogen auf das Erdvolumen). Daraufhin werden die Töpfe mit Wasser soweit gefüllt, dass die untersten Stengelteile der Reispflanzen im Wasser stehen. Nach 96 Stunden werden die behandelten Reispflanzen mit einer Konidiensuspension des Pilzes infiziert. Nach einer Inkubation der infizierten Pflanzen während 5 Tagen bei 95-100 % relativer Luftfeuchtigkeit und ca. 24 °C wird der Pilzbefall beurteilt.

Reispflanzen, die mit einer Spritzbrühe behandelt wurden, die als Aktivsubstanz eine Verbindung aus den Tabellen 1 bis 4 enthielt, zeigten im Vergleich zu unbehandelten Kontrollpflanzen (100 % Befall) nur geringen Pilzbefall. So reduzierten z.B. im Test (a) die Verbindungen 1.5, 1.7, 1.41 und 1.84 und im Test (b) die Verbindungen 1.1, 1.5, 1.7, 1.8, 1.26, 1.37, 1.85, 1.86, 1.90 und 2.1 den Pilzbefall auf 5 bis 20 %.

Beispiel 3.9: Wirkung gegen Pseudomonas tomato an Tomatenpflanzen

a) Tomatenpflanzen werden nach 3-wöchiger Anzucht mit einer aus Spritzpulver des Wirkstoffes hergestellten Spritzbrühe durch Blattapplikation behandelt (Konzentration: 200 ppm). Nach 3,5 Wochen werden die Pflanzen mit einer Bakteriensuspension ($10^8$ Bakterien/ml) inokuliert und für 6 Tage bei hoher Luftfeuchtigkeit und bei einer Temperatur von 25 °C inkubiert. Die Beurteilung der Schutzwirkung erfolgt aufgrund des Bakterienbefalls 7-8 Tage nach Inokulation.

Unbehandelte aber infizierte Kontrollpflanzen weisen in diesem Test einen Befall von 100 % auf.

Verbindungen aus den Tabellen 1 bis 4 zeigten eine gute Schutzwirkung gegen Pseudomonas tomato. So blieben Pflanzen, die z.B. mit den Verbindungen 1.7, 1.8 und 3.8 behandelt wurden, weitgehend frei von Pseudomonas (Befall: 20 bis 0 %).

b) Tomatenpflanzen werden nach 3-wöchiger Anzucht mit einer aus Spritzpulver des Wirkstoffes hergestellten Spritzbrühe durch Bodenapplikation behandelt (Konzentration: 60 ppm bezogen auf das Bodenvolumen. Nach 3,5 Wochen werden die Pflanzen mit Bakteriensuspension ($10^8$ Bakterien/ml) inokuliert und für 6 Tage bei hoher Luftfeuchtigkeit und bei einer Temperatur von 25 °C inkubiert.

Die Beurteilung der Schutzwirkung erfolgt aufgrund des Bakterienbefalls 7-8 Tage nach Inokulation.

Unbehandelte aber infizierte Kontrollpflanzen wiesen in diesem Test einen Befall von 100 % auf.

Verbindungen aus den Tabellen 1 bis 4 zeigten eine gute Schutzwirkung gegen Pseudomonas tomato. So blieben Pflanzen, die z.B. mit der Verbindung 1.7, 1.8 oder 3.8 behandelt wurden, fast völlig frei von Pseudomonas (Befall: 20 bis 0 %).

Beispiel 3.10: Wirkung gegen Tabakmosaikvirus auf Tabak

Tabakpflanzen (8 Wochen alt) werden mit einer formulierten Lösung des Wirkungsstoffes injiziert (Konzentration: 200 ppm). Nach 4 Tagen werden die Pflanzen mit einer Suspension von Tabakmosaikvirus (0.5 µg/ml + Carborundum) mechanisch inokuliert und bei einer Temperatur von 20 °-22 °C inkubiert.

Die Beurteilung der Schutzwirkung erfolgte aufgrund der Anzahl und der Grösse der Lokalläsionen 7 Tage nach der Inokulation.

Pflanzen, welche mit den Verbindungen aus Tabellen 1 bis 4 behandelt wurden, zeigten eine deutliche Verminderung der Läsionen im Vergleich mit unbehandelten jedoch infizierten Pflanzen, die einen Befall von 100 % aufwiesen.

## Patentansprüche

**Patentansprüche für folgende Vertragsstaaten : AT, BE, CH, DE, FR, GB, GR, IT, LI, LU, NL, SE**

**1.** Verbindungen der Formel I

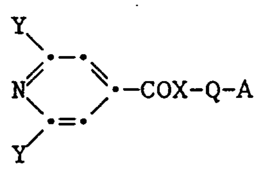

(I)

in welcher bedeuten:
Y Halogen;
X Sauerstoff oder Schwefel;
Q $C_1$-$C_3$-Alkylen, Propenylen, mit R ein- oder zweifach substituiertes $C_1$-$C_3$-Alkylen oder mit R ein- oder zweifach substituiertes Propenylen.
R $C_1$-$C_4$-Alkyl, $C_1$-$C_4$-Haloalkyl mit 1 bis 3 Halogenatomen, Cyano, $C_2$-$C_5$-Alkoxycarbonyl, $C_3$-$C_6$-Cycloalkyl, Phenyl oder mit $C_1$-$C_4$-Alkyl, $C_1$-$C_4$-Alkoxy, Trifluormethyl, Trichlormethyl, Nitro oder Cyano substituiertes Phenyl, oder Benzoyl oder mit $C_1$-$C_4$-Alkyl, $C_1$-$C_4$-Alkoxy, Trifluormethyl, Trichlormethyl, Nitro oder Cyano substituiertes Benzoyl;
A Phenyl, Biphenyl, Phenoxyphenyl, Naphthyl, Pyridyl, Furyl, Thienyl, Imidazolyl oder Triazolyl, wobei diese Reste unsubstituiert oder mit Halogen, $C_1$-$C_4$-Alkyl, $C_1$-$C_3$-Alkoxy, Trifluormethyl, Nitro oder Cyano substituiert sein können; oder R ist p-Chlorphenyl, wenn Y = Chlor, X = Sauerstoff, Q = Methylen und A = 3,4-Dimethoxyphenyl sind; mit der Massgabe, 1.) dass falls A Imidazolyl oder Triazolyl darstellt, R nicht Phenyl oder Benzoyl sein darf, und 2.) dass für A und für R in Q zusammen maximal 3 Ringe stehen dürfen.

**2.** Verbindungen der Formel I

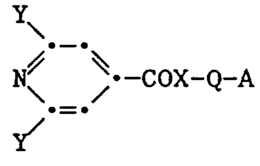

(I)

in welcher bedeuten:
Y Halogen;
X Sauerstoff oder Schwefel;
Q $C_1$-$C_3$-Alkylen, Propenylen, mit R ein- oder zweifach substituiertes $C_1$-$C_3$-Alkylen oder mit R ein- oder zweifach substituiertes Propenylen.
R $C_1$-$C_4$-Alkyl, $C_1$-$C_4$-Haloalkyl mit 1 bis 3 Halogenatomen, Cyano, $C_2$-$C_5$-Alkoxycarbonyl, $C_3$-$C_6$-Cycloalkyl, Phenyl oder mit $C_1$-$C_4$-Alkyl, $C_1$-$C_4$-Alkoxy, Trifluormethyl, Trichlormethyl, Nitro oder Cyano substituiertes Phenyl, oder Benzoyl oder mit $C_1$-$C_4$-Alkyl, $C_1$-$C_4$-Alkoxy, Trifluormethyl, Trichlormethyl, Nitro oder Cyano substituiertes Benzoyl;
A Phenyl, Biphenyl, Phenoxyphenyl, Naphthyl, Pyridyl, Furyl, Thienyl, Imidazolyl oder Triazolyl, wobei diese Reste unsubstituiert oder mit Halogen, $C_1$-$C_4$-Alkyl, $C_1$-$C_3$-Alkoxy, Trifluormethyl, Nitro oder Cyano substituiert sein können; mit der Massgabe, 1.) dass falls A Imidazolyl oder Triazolyl darstellt, R nicht Phenyl oder Benzoyl sein darf, und 2.) dass für A und für R in Q zusammen maximal 3 Ringe stehen dürfen.

**3.** Verbindungen gemäss Anspruch 2, worin bedeuten:
Y gleichzeitig Chlor oder Brom;
X Sauerstoff;
Q Methylen oder mit R substituiertes Methylen;
R $C_1$-$C_3$-Alkyl, Phenyl oder mit Halogen oder Methoxy substituiertes Phenyl;
A Phenyl, mit Halogen substituiertes Phenyl, oder Pyridyl, Furyl, Thienyl, Imidazolyl oder Triazolyl.

**4.** Verbindungen gemäss Anspruch 2, worin bedeuten:
Y Chlor;
X Sauerstoff;
Q mit R substituiertes Methylen;
R Methyl, Ethyl, Phenyl oder 2,4-Dichlorphenyl;
A mit Chlor und/oder Fluor substituiertes Phenyl, insbesondere 2,4-Dichlorphenyl.

**5.** Eine Verbindung aus der Gruppe:
2,6-Dichlor-isonicotinsäurebenzylester;
2,6-Dichlor-isonicotinsäure-α-methylbenzylester;
2,6-Dichlor-isonicotinsäure-α-ethylbenzylester;
2,6-Dichlor-isonicotinsäure-α-phenylbenzylester;
2,6-Dichlor-isonicotinsäure-α-(4-chlorphenyl)-benzylester.

**6.** Verfahren zur Herstellung der Verbindungen der Formel I in Anspruch 1, dadurch gekennzeichnet, dass man umsetzt:

a) ein Isonicotinsäurehalogenid der Formel II

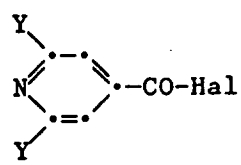

(II)

oder

b) ein Isonicotinsäureanhydrid der Formel IV

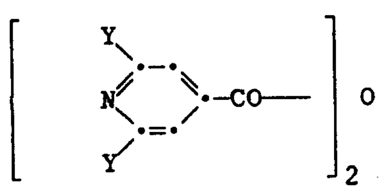

(IV)

oder

c) ein Isonicotinsäureazolid der Formel V

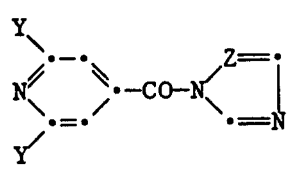

(V)

oder

d) ein Isonicotinsäurederivat der Formel VI

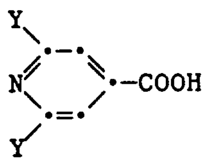

(VI)

mit einem Alkohol der Formel III

A-Q-XH (III);

wobei Z CH oder N darstellt und Y, A, Q und X die unter Formel I angegebenen Bedeutungen besitzen.

**7.** Mittel zum Schutz von Pflanzen gegen den Befall durch Mikroorganismen, dadurch gekennzeichnet, dass es neben üblichen Träger- und Hilfsstoffen als aktive Komponente mindestens eine Verbindung gemäss Anspruch 1 enthält.

**8.** Mittel gemäss Anspruch 7, dadurch gekennzeichnet, dass es als aktive Komponente mindestens eine Verbindung gemäss den Ansprüchen 2 bis 4 enthält.

**9.** Mittel gemäss Anspruch 7, dadurch gekennzeichnet, dass es als aktive Komponente mindestens eine Verbindung der Formel I gemäss Anspruch 5 enthält.

**10.** Verfahren zum Schutz von Pflanzen gegen den Befall durch phytopathogene Mikroorganismen, dadurch gekennzeichnet, dass man als Wirkstoff eine Verbindung gemäss Anspruch 1 auf die Pflanze oder deren Standort appliziert.

**11.** Verfahren zum Schutz von Pflanzen gegen den Befall durch phytopathogene Mikroorganismen, dadurch gekennzeichnet, dass man als Wirkstoff eine Verbindung gemäss einem der Ansprüche 2 bis 5 auf die Pflanze oder deren Standort appliziert.

**12.** Verwendung von Verbindungen gemäss Anspruch 1 zum Schutz von Pflanzen gegen den Befall durch phytopathogene Mikroorganismen.

**13.** Verwendung von Verbindungen gemäss einem der Ansprüche 2 bis 5 zum Schutz von Pflanzen gegen den Befall durch phytopathogene Mikroorganismen.

**Patentansprüche für folgenden Vertragsstaat : ES**

**1.** Verfahren zur Herstellung der Verbindungen der Formel I

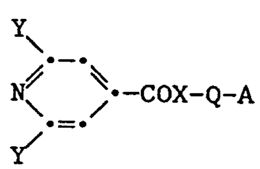

(I)

in welcher bedeuten:
Y Halogen;
X Sauerstoff oder Schwefel;
Q $C_1$-$C_3$-Alkylen, Propenylen, mit R ein- oder zweifach substituiertes $C_1$-$C_3$-Alkylen oder mit R ein- oder zweifach substituiertes Propenylen.
R $C_1$-$C_4$-Alkyl, $C_1$-$C_4$-Haloalkyl mit 1 bis 3 Halogenatomen, Cyano, $C_2$-$C_5$-Alkoxycarbonyl, $C_3$-$C_6$-Cycloalkyl, Phenyl oder mit $C_1$-$C_4$-Alkyl, $C_1$-$C_4$-Alkoxy, Trifluormethyl, Trichlormethyl, Nitro oder Cyano

substituiertes Phenyl, oder Benzoyl oder mit $C_1$-$C_4$-Alkyl, $C_1$-$C_4$-Alkoxy, Trifluormethyl, Trichlormethyl, Nitro oder Cyano substituiertes Benzoyl;
A Phenyl, Biphenyl, Phenoxyphenyl, Naphthyl, Pyridyl, Furyl, Thienyl, Imidazolyl oder Triazolyl, wobei diese Reste unsubstituiert oder ein- bis dreifach mit Halogen, $C_1$-$C_4$-Alkyl, $C_1$-$C_3$-Alkoxy, Trifluormethyl, Nitro oder Cyano substituiert sein können; oder R ist p-Chlorphenyl, wenn Y = Chlor, X = Sauerstoff, Q = Methylen und A = 3,4-Dimethoxyphenyl sind; mit der Massgabe, 1.) dass falls A Imidazolyl oder Triazolyl darstellt, R nicht Phenyl oder Benzoyl sein darf, und 2.) dass für A und für R in Q zusammen maximal 3 Ringe stehen dürfen, dadurch gekennzeichnet, dass man umsetzt:

a) ein Isonicotinsäurehalogenid der Formel II

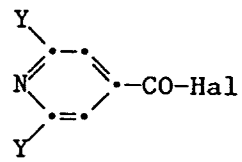

(II)

oder

b) ein Isonicotinsäureanhydrid der Formel IV

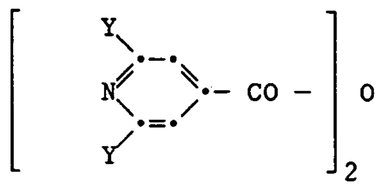

(IV)

oder

c) ein Isonicotinsäureazolid der Formel V

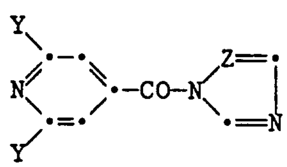

(V)

oder

d) ein Isonicotinsäurederivat der Formel VI

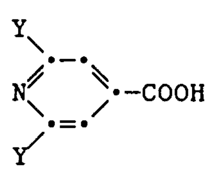

(VI)

mit einem Alkohol der Formel III

A-Q-XH (III);

wobei Z CH oder N darstellt und Y, A, Q und X die unter Formel I angegebenen Bedeutungen besitzen.

**2.** Verfahren nach Anspruch 1, dadurch gekennzeichnet, dass Verbindungen der Formel I hergestellt werden,

worin bedeuten:
Y Halogen;
X Sauerstoff oder Schwefel;
Q $C_1$-$C_3$-Alkylen, Propenylen, mit R ein- oder zweifach substituiertes $C_1$-$C_3$-Alkylen oder mit R ein- oder zweifach substituiertes Propenylen.
R $C_1$-$C_4$-Alkyl, $C_1$-$C_4$-Haloalkyl mit 1 bis 3 Halogenatomen, Cyano, $C_2$-$C_5$-Alkoxycarbonyl, $C_3$-$C_6$-Cycloalkyl, Phenyl oder mit $C_1$-$C_4$-Alkyl, $C_1$-$C_4$-Alkoxy, Trifluormethyl, Trichlormethyl, Nitro oder Cyano substituiertes Phenyl, oder Benzoyl oder mit $C_1$-$C_4$-Alkyl, $C_1$-$C_4$-Alkoxy, Trifluormethyl, Trichlormethyl, Nitro oder Cyano substituiertes Benzoyl;
A Phenyl, Biphenyl, Phenoxyphenyl, Naphthyl, Pyridyl, Furyl, Thienyl, Imidazolyl oder Triazolyl, wobei diese Reste unsubstituiert oder mit Halogen, $C_1$-$C_4$-Alkyl, $C_1$-$C_3$-Alkoxy, Trifluormethyl, Nitro oder Cyano substituiert sein können; mit der Massgabe, 1.) dass falls A Imidazolyl oder Triazolyl darstellt, R nicht Phenyl oder Benzoyl sein darf, und 2.) dass für A und für R in Q zusammen maximal 3 Ringe stehen dürfen.

3. Verfahren nach Anspruch 1, dadurch gekennzeichnet, dass Verbindungen der Formel I hergestellt werden, worin bedeuten:
Y gleichzeitig Chlor oder Brom;
X Sauerstoff;
Q Methylen oder mit R substituiertes Methylen;
R $C_1$-$C_3$-Alkyl, Phenyl oder mit Halogen oder Methoxy substituiertes Phenyl;
A Phenyl, mit Halogen substituiertes Phenyl, oder Pyridyl, Furyl, Thienyl, Imidazolyl oder Triazolyl.

4. Verfahren nach Anspruch 1, dadurch gekennzeichnet, dass Verbindungen der Formel I hergestellt werden, worin bedeuten:
Y Chlor;
X Sauerstoff;
Q mit R substituiertes Methylen;
R Methyl, Ethyl, Phenyl oder 2,4-Dichlorphenyl;
A mit Chlor und/oder Fluor substituiertes Phenyl, insbesondere 2,4-Dichlorphenyl.

5. Verfahren nach Anspruch 1, dadurch gekennzeichnet, dass eine Verbindung hergestellt wird aus der Gruppe:
2,6-Dichlor-isonicotinsäurebenzylester;
2,6-Dichlor-isonicotinsäure-α-methylbenzylester;
2,6-Dichlor-isonicotinsäure-α-ethylbenzylester;
2,6-Dichlor-isonicotinsäure-α-phenylbenzylester;
2,6-Dichlor-isonicotinsäure-α-(4-chlorphenyl)-benzylester.

## Claims

**Claims for the following Contracting States : AT, BE, CH, DE, FR, GB, GR, IT, LI, LU, NL, SE**

1. A compound of formula I

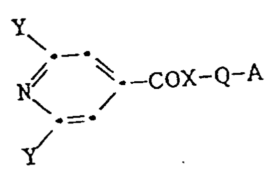

(I)

in which
Y is halogen;
X is oxygen or sulfur;
Q is $C_1$-$C_3$alkylene, propenylene, $C_1$-$C_3$alkylene mono- or di-substituted by R, or propenylene mono- or di-substituted by R;
R is $C_1$-$C_4$alkyl, $C_1$-$C_4$haloalkyl having from 1 to 3 halogen atoms, cyano, $C_2$-$C_5$alkoxycarbonyl, $C_3$-$C_6$cycloalkyl, phenyl, or phenyl substituted by $C_1$-$C_4$alkyl, $C_1$-$C_4$alkoxy, trifluoromethyl, trichloromethyl,

nitro or by cyano, or benzoyl or benzoyl substituted by $C_1$-$C_4$alkyl, $C_1$-$C_4$alkoxy, trifluoromethyl, trichloromethyl, nitro or by cyano;
A is phenyl, biphenyl, phenoxyphenyl, naphthyl, pyridyl, furyl, thienyl, imidazolyl or triazolyl, it being possible for each of these radicals to be unsubstituted or substituted by halogen, $C_1$-$C_4$alkyl, $C_1$-$C_3$alkoxy, trifluoromethyl, nitro or by cyano;
or R is p-chlorophenyl when Y is chlorine, X is oxygen, Q is methylene and A is 3,4-dimethoxyphenyl;
with the proviso 1.) that if A is imidazolyl or triazolyl R may not be phenyl or benzoyl, and 2.) that A and the R substituent in Q may together contain no more than 3 rings.

**2.** A compound of formula I

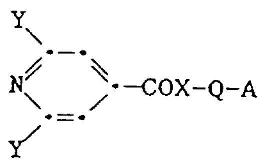

(I)

in which:
Y is halogen;
X is oxygen or sulfur;
Q is $C_1$-$C_3$alkylene, propenylene, $C_1$-$C_3$alkylene mono- or di-substituted by R, or propenylene mono- or di-substituted by R;
R is $C_1$-$C_4$alkyl, $C_1$-$C_4$haloalkyl having from 1 to 3 halogen atoms, cyano, $C_2$-$C_5$alkoxycarbonyl, $C_3$-$C_6$cycloalkyl, phenyl, or phenyl substituted by $C_1$-$C_4$alkyl, $C_1$-$C_4$alkoxy, trifluoromethyl, trichloromethyl, nitro or by cyano, or benzoyl or benzoyl substituted by $C_1$-$C_4$alkyl, $C_1$-$C_4$alkoxy, trifluoromethyl, trichloromethyl, nitro or by cyano;
A is phenyl, biphenyl, phenoxyphenyl, naphthyl, pyridyl, furyl, thienyl, imidazolyl or triazolyl, it being possible for each of these radicals to be unsubstituted or substituted by halogen, $C_1$-$C_4$alkyl, $C_1$-$C_3$alkoxy, trifluoromethyl, nitro or by cyano; with the proviso 1.) that if A is imidazolyl or triazolyl R may not be phenyl or benzoyl, and 2.) that A and the R substituent in Q may together contain no more than 3 rings.

**3.** A compound according to claim 2 in which:
Y is simultaneously chlorine or bromine;
X is oxygen;
Q is methylene or methylene substituted by R;
R is $C_1$-$C_3$alkyl, phenyl or phenyl substituted by halogen or by methoxy;
A is phenyl, phenyl substituted by halogen, or pyridyl, furyl, thienyl, imidazolyl or triazolyl.

**4.** A compound according to claim 2 in which:
Y is chlorine;
X is oxygen;
Q is methylene substituted by R;
R is methyl, ethyl, phenyl or 2,4-dichlorophenyl;
A is phenyl substituted by chlorine and/or by fluorine, especially 2,4-dichlorophenyl.

**5.** A compound from the group:
2,6-dichloroisonicotinic acid benzyl ester;
2,6-dichloroisonicotinic acid $\alpha$-methylbenzyl ester;
2,6-dichloroisonicotinic acid $\alpha$-ethylbenzyl ester;
2,6-dichloroisonicotinic acid $\alpha$-phenylbenzyl ester;
2,6-dichloroisonicotinic acid $\alpha$-(4-chlorophenyl)-benzyl ester.

**6.** A process for the preparation of a compound of formula I in claim 1 which comprises reacting:

a) an isonicotinic acid halide of formula II

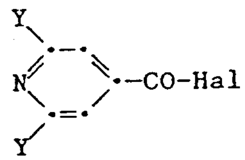

(II)

or

b) an isonicotinic acid anhydride of formula IV

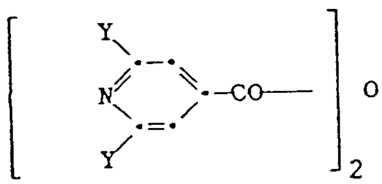

(IV)

or

c) an isonicotinic acid azolide of formula V

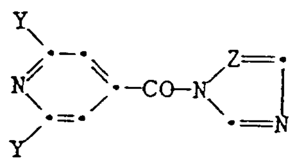

(V)

or

d) an isonicotinic acid derivative of formula VI

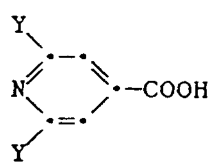

(VI)

with an alcohol of formula III

A-Q-XH (III);

wherein Z is CH or N and Y, A, Q and X are as defined for formula I.

**7.** A composition for protecting plants against attack by microorganisms that comprises at least one compound according to claim 1 as active component together with customary carriers and adjuvants.

**8.** A composition according to claim 7 that comprises at least one compound according to claims 2 to 4 as active component.

**9.** A composition according to claim 7 that comprises at least one compound of formula I according to claim 5 as active component.

**10.** A method of protecting plants against attack by phytopathogenic microorganisms which comprises applying as active ingredient to the plant or to the locus thereof a compound according to claim 1.

**11.** A method of protecting plants against attack by phytopathogenic microorganisms which comprises applying as active ingredient to the plant or to the locus thereof a compound according to any one of claims 2 to 5.

**12.** The use of a compound according to claim 1 for protecting plants against attack by phytopathogenic microorganisms.

**13.** The use of a compound according to any one of claims 2 to 5 for protecting plants against attack by phytopathogenic microorganisms.

**Claims for the following Contracting State : ES**

**1.** A process for the preparation of a compound of formula I

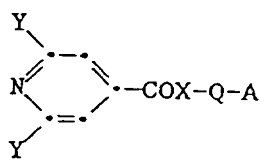

(I)

in which
Y is halogen;
X is oxygen or sulfur;
Q is $C_1$-$C_3$alkylene, propenylene, $C_1$-$C_3$alkylene mono- or di-substituted by R, or propenylene mono- or di-substituted by R;
R is $C_1$-$C_4$alkyl, $C_1$-$C_4$haloalkyl having from 1 to 3 halogen atoms, cyano, $C_2$-$C_5$alkoxycarbonyl, $C_3$-$C_6$cycloalkyl, phenyl, or phenyl substituted by $C_1$-$C_4$alkyl, $C_1$-$C_4$alkoxy, trifluoromethyl, trichloromethyl, nitro or by cyano, or benzoyl or benzoyl substituted by $C_1$-$C_4$alkyl, $C_1$-$C_4$alkoxy, trifluoromethyl, trichloromethyl, nitro or by cyano;
A is phenyl, biphenyl, phenoxyphenyl, naphthyl, pyridyl, furyl, thienyl, imidazolyl or triazolyl, it being possible for each of these radicals to be unsubstituted or mono- or tri-substituted by halogen, $C_1$-$C_4$alkyl, $C_1$-$C_3$alkoxy, trifluoromethyl, nitro or by cyano;
or R is p-chlorophenyl when Y is chlorine, X is oxygen, Q is methylene and A is 3,4-dimethoxyphenyl;
with the proviso 1.) that if A is imidazolyl or triazolyl R may not be phenyl or benzoyl, and 2.) that A and the R substituent in Q may together contain no more than 3 rings, which comprises reacting:

a) an isonicotinic acid halide of formula II

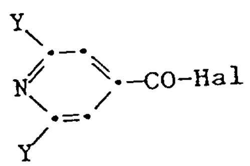

(II)

or

b) an isonicotinic acid anhydride of formula IV

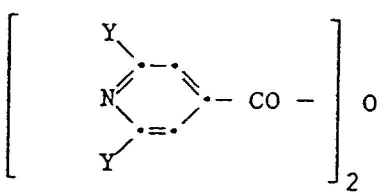

(IV)

or

c) an isonicotinic acid azolide of formula V

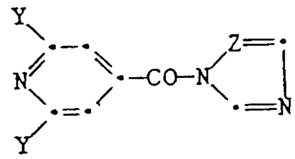

(V)

or

d) an isonicotinic acid derivative of formula VI

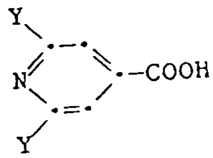

(VI)

with an alcohol of formula III

A-Q-XH (III);

wherein Z is CH or N and Y, A, Q and X are as defined for formula I.

**2.** A process according to claim 1 which comprises the preparation of a compound of formula I in which:
Y is halogen;
X is oxygen or sulfur;
Q is $C_1$-$C_3$alkylene, propenylene, $C_1$-$C_3$alkylene mono- or di-substituted by R, or propenylene mono- or di-substituted by R;
R is $C_1$-$C_4$alkyl, $C_1$-$C_4$haloalkyl having from 1 to 3 halogen atoms, cyano, $C_2$-$C_5$alkoxycarbonyl, $C_3$-$C_6$cycloalkyl, phenyl, or phenyl substituted by $C_1$-$C_4$alkyl, $C_1$-$C_4$alkoxy, trifluoromethyl, trichloromethyl, nitro or by cyano, or benzoyl or benzoyl substituted by $C_1$-$C_4$alkyl, $C_1$-$C_4$alkoxy, trifluoromethyl, trichloromethyl, nitro or by cyano;
A is phenyl, biphenyl, phenoxyphenyl, naphthyl, pyridyl, furyl, thienyl, imidazolyl or triazolyl, it being possible for each of these radicals to be unsubstituted or substituted by halogen, $C_1$-$C_4$alkyl, $C_1$-$C_3$alkoxy, trifluoromethyl, nitro or by cyano; with the proviso 1.) that if A is imidazolyl or triazolyl R may not be phenyl or benzoyl, and 2.) that A and the R substituent in Q may together contain no more than 3 rings.

**3.** A process according to claim 1 which comprises the preparation of a compound of formula I in which:
Y is simultaneously chlorine or bromine;
X is oxygen;
Q is methylene or methylene substituted by R;
R is $C_1$-$C_3$alkyl, phenyl or phenyl substituted by halogen or by methoxy;
A is phenyl, phenyl substituted by halogen, or pyridyl, furyl, thienyl, imidazolyl or triazolyl.

**4.** A process according to claim 1 which comprises the preparation of a compound of formula I in which:
Y is chlorine;
X is oxygen;
Q is methylene substituted by R;
R is methyl, ethyl, phenyl or 2,4-dichlorophenyl;
A is phenyl substituted by chlorine and/or by fluorine, especially 2,4-dichlorophenyl.

**5.** A process according to claim 1 which comprises the preparation of a compound from the group:
2,6-dichloroisonicotinic acid benzyl ester;

2,6-dichloroisonicotinic acid α-methylbenzyl ester;
2,6-dichloroisonicotinic acid α-ethylbenzyl ester;
2,6-dichloroisonicotinic acid α-phenylbenzyl ester;
2,6-dichloroisonicotinic acid α-(4-chlorophenyl)-benzyl ester.

## Revendications

**Revendications pour les Etats contractants suivants : AT, BE, CH, DE, FR, GB, GR, IT, LI, LU, NL, SE**

**1.** Composés de formule I

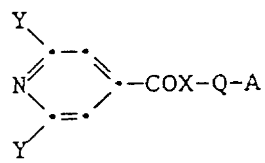

(I)

dans laquelle

Y représente un halogène;

X représente l'oxygène ou le soufre;

Q représente un groupe alkyle en C 1-C 3, un groupe propénylène, un groupe alkylène en C 1-C 3 portant un ou deux substituants R ou un groupe propénylène portant un ou deux substituants R,

R représente un groupe alkyle en C 1-C 4, halogénoalkyle contenant 1 à 4 atomes de carbone et 1 à 3 atomes d'halogènes, cyano, alcoxycarbonyle en C 2-C 5, cycloalkyle en C 3-C 6, phényle ou phényle substitué par des groupes alkyle en C 1-C 4, alcoxy en C 1-C 4, trifluorométhyle, trichlorométhyle, nitro ou cyano, ou un groupe benzoyle ou benzoyle substitué par des groupes alkyle en C 1-C 4, alcoxy en C 1-C 4, trifluorométhyle, trichlorométhyle, nitro ou cyano;

A représente un groupe phényle, biphényle, phénoxyphényle, naphtyle, pyridyle, furyle, thiényle, imidazolyle ou triazolyle, ces groupes pouvant être non substitués ou substitués par des halogènes, des groupes alkyle en C 1-C 4, alcoxy en C 1-C 3, trifluorométhyle, nitro ou cyano; ou bien R représente un groupe p-chlorophényle lorsque Y représente le chlore, X l'oxygène, Q un groupe méthylène et A un groupe 3,4-diméthoxyphényle; sous réserve 1) que lorsque A représente un groupe imidazolyle ou triazolyle, R ne peut représenter un groupe phényle ou benzoyle, et 2) que pour A et pour R dans Q, ensemble, il y a au maximum 3 cycles.

**2.** Composés de formule I

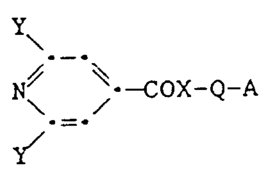

(I)

dans laquelle

Y représente un halogène;

X représente l'oxygène ou le soufre;

Q représente un groupe alkylène en C 1-C 3, propénylène, alkylène en C 1-C 3 portant un ou deux substituants R ou propénylène portant un ou deux substituants R;

R représente un groupe alkyle en C 1-C 4, halogénoalkyle contenant 1 à 4 atomes de carbone et 1 à 3 atomes d'halogènes, cyano, alcoxycarbonyle en C 2-C 5, cycloalkyle en C 3-C 6, phényle ou phényle substitué par des groupes alkyle en C 1-C 4, alcoxy en C 1-C 4, trifluorométhyle, trichlorométhyle, nitro ou cyano, ou bien un groupe benzoyle ou un groupe benzoyle substitué par des groupes alkyle en C 1-C 4, alcoxy en C 1-C 4, trifluorométhyle,

trichlorométhyle, nitro ou cyano;

A représente un groupe phényle, biphényle, phénoxyphényle, naphtyle, pyridyle, furyle, thiényle, imidazolyle ou triazolyle, ces groupes pouvant être non substitués ou substitués par des halogènes, des groupes alkyle en C 1-C 4, alcoxy en C 1-C 3, trifluorométhyle, nitro ou cyano; sous réserve 1) que lorsque A représente un groupe imidazolyle ou triazolyle, R ne peut représenter un groupe phényle ou benzoyle, et 2) que pour A et pour R dans Q, ensemble, il y a au maximum 3 cycles.

**3.** Composés selon revendication 2, pour lesquels :
les symboles Y représentent tous deux le chlore ou tous deux le brome;
X représente l'oxygène;
Q représente un groupe méthylène ou méthylène substitué par R;
R représente un groupe alkyle en C 1-C 3, phényle ou phényle substitué par un halogène ou un groupe méthoxy;
A représente un groupe phényle, phényle substitué par un halogène, ou pyridyle, furyle, thiényle, imidazolyle ou triazolyle.

**4.** Composés selon revendication 2, pour lesquels :
Y représente le chlore;
X représente l'oxygène;
Q représente un groupe méthylène substitué par R;
R représente un groupe méthyle, éthyle, phényle ou 2,4-dichlorophényle;
A représente un groupe phényle substitué par le chlore et/ou le fluor, en particulier un groupe 2,4-dichlorophényle.

**5.** Un composé du groupe suivant :
2,6-dichlorisonicotinate de benzyle;
2,6-dichlorisonicotinate d'alpha-méthylbenzyle;
2,6-dichlorisonicotinate d'alpha-éthylbenzyle;
2,6-dichlorisonicotinate d'alpha-phénylbenzyle;
2,6-dichlorisonicotinate d'alpha-(4-chlorophényl)-benzyle.

**6.** Procédé de préparation des composés de formule I de la revendication 1, caractérisé en ce que l'on fait réagir :

a) un halogénure d'acide isonicotinique de formule II

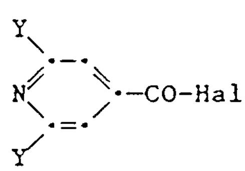

(II)

ou bien

b) un anhydride d'acide isonicotinique de formule IV

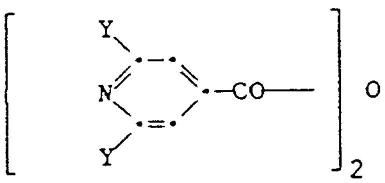

(IV)

ou bien

c) un azolide d'acide isonicotinique de formule V

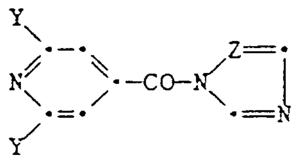

(V)

ou bien

d) un dérivé d'acide isonicotinique de formule VI

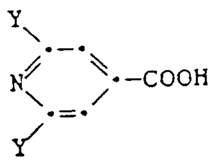

(VI)

avec un alcool de formule III

A-Q-XH (III)

Z représentant CH ou N, et Y, A, Q et X ayant les significations indiquées en référence à la formule I.

**7.** Produit pour protéger les végétaux contre l'attaque par des microorganismes caractérisé en ce qu'il contient, avec des véhicules et produits auxiliaires usuels, au moins un composant actif consistant en un composé selon revendication 1.

**8.** Produit selon revendication 7, caractérisé en ce qu'il contient au moins un composant actif consistant en un composé selon les revendications 2 à 4.

**9.** Produit selon revendication 7, caractérisé en ce qu'il contient au moins un composant actif consistant en un composé de formule I de la revendication 5.

**10.** Procédé pour protéger les végétaux contre une attaque par des microorganismes phytopathogènes, caractérisé en ce que l'on applique sur la plante ou son habitat une substance active consistant en un composé selon revendication 1.

**11.** Procédé pour protéger les végétaux contre une attaque par des microorganismes phytopathogènes, caractérisé en ce que l'on applique sur la plante ou son habitat une substance active consistant en un composé selon l'une des revendications 2 à 5.

**12.** Utilisation des composés selon revendication 1 pour la protection des végétaux contre une attaque par des microorganismes phytopathogènes.

**13.** Utilisation des composés selon l'une des revendications 2 à 5 pour la protection des végétaux contre une attaque par des microorganismes phytopathogènes.

**Revendications pour l'Etat contractant suivant : ES**

**1.** Procédé de préparation des composés de formule I

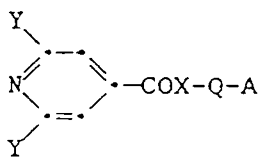

(I)

dans laquelle

Y représente un halogène;

X représente l'oxygène ou le soufre;

Q représente un groupe alkyle en C 1-C 3, un groupe propénylène, un groupe alkylène en C 1-C 3 portant un ou deux substituants R ou un groupe propénylène portant un ou deux substituants R,

R représente un groupe alkyle en C 1-C 4, halogénoalkyle contenant 1 à 4 atomes de carbone et 1 à 3 atomes d'halogènes, cyano, alcoxycarbonyle en C 2-C 5, cycloalkyle en C 3-C 6, phényle ou phényle substitué par des groupes alkyle en C 1-C 4, alcoxy en C 1-C 4, trifluorométhyle, trichlorométhyle, nitro ou cyano, ou un groupe benzoyle ou benzoyle substitué par des groupes alkyle en C 1-C 4, alcoxy en C 1-C 4, trifluorométhyle, trichlorométhyle, nitro ou cyano;

A représente un groupe phényle, biphényle, phénoxyphényle, naphtyle, pyridyle, furyle, thiényle, imidazolyle ou triazolyle, ces groupes pouvant être non substitués ou substitués par des halogènes, des groupes alkyle en C 1-C 4, alcoxy en C 1-C 3, trifluorométhyle, nitro ou cyano; ou bien R représente un groupe p-chlorophényle lorsque Y représente le chlore, X l'oxygène, Q un groupe méthylène et A un groupe 3,4-diméthoxyphényle; sous réserve 1) que lorsque A représente un groupe imidazolyle ou triazolyle, R ne peut représenter un groupe phényle ou benzoyle, et 2) que pour A et pour R dans Q, ensemble, il y a au maximum 3 cycles,

caractérisé en ce que l'on fait réagir :

a) un halogénure d'acide isonicotinique de formule II

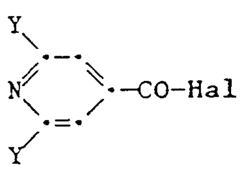

(II)

ou bien

b) un anhydride d'acide isonicotinique de formule IV

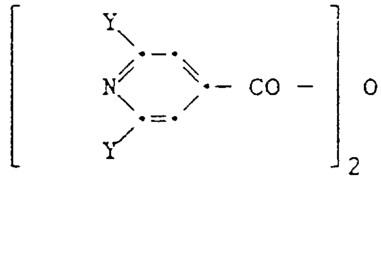

(IV)

ou bien

c) un azolide d'acide isonicotinique de formule V

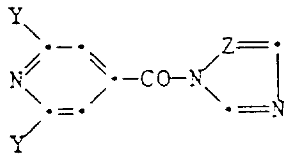

(V)

ou bien
d) un dérivé d'acide isonicotinique de formule VI

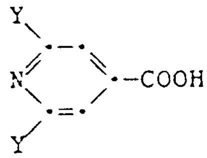

(VI)

avec un alcool de formule III

A-Q-XH (III)

Z représentant CH ou N, et Y, A, Q et X ayant les significations indiquées en référence à la formule I.

2. Procédé selon revendication 1, caractérisé en ce que l'on prépare des composés de formule I dans laquelle :
   - Y représente un halogène;
   - X représente l'oxygène ou le soufre;
   - Q représente un groupe alkylène en C 1-C 3, propénylène, alkylène en C 1-C 3 portant un ou deux substituants R ou propénylène portant un ou deux substituants R;
   - R représente un groupe alkyle en C 1-C 4, halogénoalkyle contenant 1 à 4 atomes de carbone et 1 à 3 atomes d'halogènes, cyano, alcoxycarbonyle en C 2-C 5, cycloalkyle en C 3-C 6, phényle ou phényle substitué par des groupes alkyle en C 1-C 4, alcoxy en C 1-C 4, trifluorométhyle, trichlorométhyle, nitro ou cyano, ou bien un groupe benzoyle ou un groupe benzoyle substitué par des groupes alkyle en C 1-C 4, alcoxy en C 1-C 4, trifluorométhyle, trichlorométhyle, nitro ou cyano;
   - A représente un groupe phényle, biphényle, phénoxyphényle, naphtyle, pyridyle, furyle, thiényle, imidazolyle ou triazolyle, ces groupes pouvant être non substitués ou substitués par des halogènes, des groupes alkyle en C 1-C 4, alcoxy en C 1-C 3, trifluorométhyle, nitro ou cyano; sous réserve 1) que lorsque A représente un groupe imidazolyle ou triazolyle, R ne peut représenter un groupe phényle ou benzoyle, et 2) que pour A et pour A dans Q, ensemble, il y a au maximum 3 cycles.

3. Procédé selon revendication 1, caractérisé en ce que l'on prépare des composés de formule I dans laquelle :
   les symboles Y représentent tous deux le chlore ou tous deux le brome;
   - X représente l'oxygène;
   - Q représente un groupe méthylène ou méthylène substitué par R;
   - R représente un groupe alkyle en C 1-C 3, phényle ou phényle substitué par un halogène ou un groupe méthoxy;
   - A représente un groupe phényle, phényle substitué par un halogène, ou pyridyle, furyle, thiényle, imidazolyle ou triazolyle.

**4.** Procédé selon revendication 1, caractérisé en ce que l'on prépare des composés de formule I dans laquelle :

- Y représente le chlore;
- X représente l'oxygène;
- Q représente un groupe méthylène substitué par R;
- R représente un groupe méthyle, éthyle, phényle ou 2,4-dichlorophényle;
- A représente un groupe phényle substitué par le chlore et/ou le fluor, en particulier un groupe 2,4-dichlorophényle.

**5.** Procédé selon revendication 1, caractérisé en ce que l'on prépare un composé du groupe suivant :
2,6-dichlorisonicotinate de benzyle;
2,6-dichlorisonicotinate d'alpha-méthylbenzyle;
2,6-dichlorisonicotinate d'alpha-éthylbenzyle;
2,6-dichlorisonicotinate d'alpha-phénylbenzyle;
2,6-dichlorisonicotinate d'alpha-(4-chlorophényl)-benzyle.